# EUROPÄISCHE PATENTSCHRIFT

(11) **EP 3 801 688 B1**
(45) Veröffentlichungstag und Bekanntmachung des Hinweises auf die Patenterteilung: **18.02.2026**
(21) Anmeldenummer: 19726696.8
(22) Anmeldetag: 28.05.2019
(51) Int. Cl.: A61M 5/20, A61M 5/24, A61M 5/31

(54) **VORRICHTUNG UND VERFAHREN ZUR ABGABE MINDESTENS EINER SUBSTANZ**
DEVICE AND METHOD FOR DISPENSING AT LEAST ONE SUBSTANCE
DISPOSITIF ET PROCÉDÉ D'ADMINISTRATION D'AU MOINS UNE SUBSTANCE

(30) Priorität: 29.05.2018 EP 18174940
(43) Veröffentlichungstag der Anmeldung: 14.04.2021
(73) Patentinhaber: SHL Medical AG, 6302 Zug (CH)
(72) Erfinder: EGLOFF, Christoph, 9104 Waldstatt (CH)
(86) Internationale Anmeldenummer: PCT/EP2019/063710
(87) Internationale Veröffentlichungsnummer: WO 2019/229010

(56) Entgegenhaltungen:
- WO-A1-2017/085538
- WO-A1-86/06965
- US-A- 5 298 023
- US-A1- 2014 049 041
- US-A1- 2017 136 183

## Beschreibung

### TECHNISCHES GEBIET

Die vorliegende Erfindung betrifft eine Vorrichtung und ein Verfahren zur Abgabe mindestens einer Substanz, insbesondere an einen Patienten. Genauer gesagt betrifft die vorliegende Erfindung insbesondere eine Vorrichtung und ein Verfahren zur Autoinjektion mindestens einer Substanz durch den Anwender selbst, in der Regel also durch den Patienten, wobei die erfindungsgemäße Vorrichtung auch als Autoinjektor und das erfindungsgemäße Verfahren als Autoinjektion mittels der erfindungsgemäßen Vorrichtung, d.h. mittels des Autoinjektors bezeichnet werden kann. Ein Autoinjektor entspricht hierbei üblicherweise einem medizinischen Instrument, das zur Verabreichung einer in der Regel in flüssiger Formulierung/Darreichungsform vorliegenden medizinischen Substanz durch parenterale Injektion dient. Autoinjektoren kommen herkömmlicherweise bei einer Behandlung durch Injektion der medizinischen Substanz unter solchen Umständen zur Anwendung, bei denen die Injektion eines pharmazeutisch aktiven Wirkstoffes auch in Abwesenheit eines Arztes oder eines anderen Angehörigen der Gesundheitsberufe gewünscht ist, z.B. in Notfall-Situationen, in denen der zu injizierende Wirkstoff möglichst rasch verabreicht werden soll. Bei der Verwendung eines Autoinjektors erfolgt die Injektion üblicherweise durch den Anwender/Patienten selbst, kann aber auch durch einen Dritten erfolgen, beispielsweise für den Fall, dass der Patient selbst nicht mehr in der Lage ist, den Autoinjektor an sich anzuwenden.

### HINTERGRUND DER ERFINDUNG

Bei der Verabreichung flüssiger Formulierungen medizinischer Substanzen bzw. pharmazeutischer Wirkstoffe ist es in den meisten Fällen erforderlich, wohl definierte Substanzmengen an einen Patienten abzugeben. Häufig müssen diese Substanzen, üblicherweise Arzneimittel, dabei in den Körper des Patienten injiziert werden. Zur parenteralen Injektion kommen üblicherweise Injektionsspritzen, Arzneimittelstifte (sogenannte Pens) oder Arzneimittelpumpen zum Einsatz. Es gibt jedoch auch viele Notfall-Situationen, in denen es wünschenswert ist, einen Patienten mit einem flüssigen Medikament oder einer flüssigen Wirkstofflösung zu behandeln. Auch wenn es aufgrund der Umstände in einer solchen Situation nicht möglich sein sollte, eine intravenöse Injektion auszuführen, kann es essentiell wichtig sein, eine Wirkstofflösung möglichst rasch per Injektion zu verabreichen. Dabei unterscheiden sich jedoch die verschiedenen Substanzen, die je nach Art des Notfalls injiziert werden sollen, meist wesentlich voneinander. Beispielsweise kann handelsübliches Insulin bereits in flüssiger Formulierung über längere Zeit gelagert, durch den Anwender mit sich geführt und dann bei Bedarf ohne nennenswerte Vorbereitung direkt in den Körper injiziert werden, wohingegen an Präparate, die nicht über einen längeren Zeitraum in flüssiger Darreichungsform gelagert werden können, erhöhte Anforderungen vor oder bei der Verabreichung gestellt werden. So sind insbesondere biotechnologisch hergestellte Wirkstoffe oft nur in gefriergetrockneter bzw. lyophilisierter Form über einen längeren Zeitraum haltbar, können aber aufgrund ihrer meist pulvrigen Feststoffform nicht durch parenterale Injektion verabreicht werden, sondern müssen vor Verabreichung in eine einspritzbare flüssige Form gebracht werden. Derartige Lyophilisate werden erst kurz vor deren Verabreichung in einer Injektionslösung, beispielsweise einer wässrigen Kochsalzlösung, gelöst und dann beispielsweise in eine Spritze aufgezogen und nachfolgend injiziert. Allerdings kann ein derartiges Vorgehen auch bei Präparaten erforderlich sein, die ein Vermischen zweier Flüssigkeiten erfordern. Bei einer Notfallanwendung besteht jedoch meist das Problem, dass der Anwender entweder bereits in einem Schockzustand ist, oder aber nicht viel Zeit verlieren darf, um sich den Wirkstoff zu verabreichen. Entsprechend können ein Aufbereiten der Injektionslösung mit dem Lyophilisat von Hand, ein anschließendes Aufziehen der Lösung mit einer Spritze und das nachfolgende Injizieren der Lösung bereits zu lange dauern oder aber zu komplexe Handlungen erfordern, die letztendlich der Injektion mit dem oft lebenswichtigen Wirkstoff im Weg stehen.

Als Lösung zu dem oben stehenden Problem werden schon seit längerer Zeit automatische Injektionsvorrichtungen bzw. Autoinjektoren verwendet, die vor ihrer Anwendung in der Regel sicher aufbewahrt und durch den Anwender/Patienten mit sich geführt werden können, meist sicher und einfach zu handhaben sind und üblicherweise die voreingestellte richtige Dosis der zu injizierenden Substanz in flüssiger Formulierung für den jeweiligen Notfall bzw. abgestimmt auf das jeweilige Krankheitsbilds des Anwenders enthalten. So beschreibt beispielsweise die WO 1986/006965 A1 eine automatische Injektionseinrichtung, mit der ein Patient die Rekonstitution und Verabreichung eines Lyophilisats selbst durchführen kann. Die Funktionsweise der darin beschriebenen Injektionseinrichtung ist derart, dass ein Verdünnungsmittel mit einem trockenen Medikamentenbestandteil durch eine vorbestimmte manuelle Betätigungsprozedur gemischt wird und anschließend eine zweite manuelle Betätigungsprozedur durchgeführt werden muss, damit eine Injektionsnadel in das Muskelgewebe des Patienten eingebracht und das verflüssigte Medikament durch die Injektionsnadel in das Muskelgewebe eingespritzt wird. Ein darin beschriebenes Ausführungsbeispiel zeigt insbesondere eine Anordnung zweier Karpulen mit jeweiligen federbetätigten Stopfen, wobei durch Drehen eines Sicherheitsschlüssels und zusätzlichem Lösen eines ersten Stifts der Stopfen der ersten Karpule eine Flüssigkeit in die andere Karpule befördert, in welcher die Flüssigkeit dann durch Schütteln mit dem Lyophilisat vermischt wird. Durch ein Lösen eines zweiten Stiftes wird anschließend der Stopfen der zweiten Karpule durch die Feder bewegt und dadurch das Gemisch bzw. die Lösung aus Flüssigkeit und Lyophilisat durch die Injektionsnadel, die durch dieselbe Betätigungshandlung freigelegt wird, ausgegeben. Die hier beschriebene Injektionseinrichtung mit zwei separaten Karpulen hat aber unter anderem den entscheidenden Nachteil, dass die beiden Karpulen im Wesentlichen bereits miteinander in Verbindung stehen und dadurch ein steriler Transport des Lyophilisats innerhalb der Injektionseinrichtung nicht mehr gegeben ist. Zudem können die beiden Karpulen vor einer Anwendung der Injektionseinrichtung nicht separat voneinander unter Wahrung der Sterilität des jeweiligen Karpuleninhalts ausgetauscht oder ersetzt werden. Ein Ablauf der Haltbarkeitsdauer entweder des Lyophilisats oder aber des Verdünnungsmittels hat bei der beschriebenen Injektionseinrichtung entsprechend einen vollständigen Austausch der gesamten Injektionseinrichtung zur Folge.

Angesichts der oben genannten Probleme des bekannten Stands der Technik ist es daher die Aufgabe der vorliegenden Erfindung, eine Vorrichtung sowie ein entsprechendes Verfahren zur Abgabe mindestens einer Substanz bereitzustellen, die die Nachteile des bekannten Stands der Technik überwinden, vorzugsweise bei vereinfachter Handhabung. Dies bezieht sich insbesondere auf ein automatisches Mischen zweier Substanzen, die sich in zwei unterschiedlichen Behältern unter Wahrung der jeweiligen Sterilität befinden. Allerdings soll je nach Ausführung auch die gesamte Handhabbarkeit der Vorrichtung bei der Autoinjektion der Substanz vereinfacht werden, wobei die Autoinjektion durch kombiniertes Auslösen verschiedener Kolbenfedern schmerzfreier, schonender und sicherer werden soll.

### ZUSAMMENFASSUNG DER ERFINDUNG

Die vorhergenannten Probleme des Stands der Technik werden gemäß der vorliegenden Erfindung anhand einer Vorrichtung zur Abgabe einer Substanz sowie anhand eines entsprechenden Verfahrens gelöst, wie sie in den beigefügten Ansprüchen beschrieben sind.

Genauer gesagt stellt die vorliegende Erfindung eine Vorrichtung zur Abgabe mindestens einer Substanz bereit, wobei die abzugebende Substanz aus einer Mischung zweier anderer Substanzen besteht, nämlich einer ersten Substanz, die mindestens einen pharmazeutisch aktiven Wirkstoff enthält und einer zweiten Substanz, die in flüssiger Form vorliegt und nach Mischung mit der ersten Substanz eine injizierbare Substanz ausbildet.

Bei der ersten Substanz kann es sich dabei um einen reinen pharmazeutisch aktiven Wirkstoff oder um ein Gemisch aus einem reinen pharmazeutisch aktiven Wirkstoff und einer oder mehrerer weiterer chemischer (insbesondere pharmazeutisch akzeptabler) Verbindungen handeln, wobei die erste Substanz in flüssiger oder fester Form vorliegen kann. Vorzugsweise liegt die erste Substanz in fester Form, insbesondere in amorpher Form, vor. Besonders bevorzugt ist ein Lyophilisat. Die erste Substanz kann optional einen oder mehrere weitere pharmazeutisch aktive Wirkstoffe enthalten. Es ist bevorzugt, dass die erste Substanz keinen weiteren pharmazeutisch aktiven Wirkstoff enthält.

Bei der zweiten Substanz kann es sich um eine Flüssigkeit handeln, die aus nur einer (insbesondere pharmazeutisch akzeptablen) chemischen Verbindung besteht, oder um eine Flüssigkeit oder eine Emulsion, die aus einer Mischung zweier oder mehrerer (insbesondere pharmazeutisch akzeptabler) chemischer Verbindungen besteht, wie insbesondere eine Lösung, beispielsweise eine wässrige Kochsalzlösung oder dergleichen. Wenn es sich bei der zweiten Substanz um eine Flüssigkeit handelt, wird die zweite Substanz auch als Lösungsflüssigkeit bezeichnet. Besonders bevorzugt ist eine Rekonstitutions-Iösung zur Auflösung eines Lyophilisats. Die zweite Substanz kann optional einen oder mehrere weitere pharmazeutisch aktive Wirkstoffe enthalten. Es ist bevorzugt, dass die zweite Substanz keinen weiteren pharmazeutisch aktiven Wirkstoff enthält.

Die durch Mischen der ersten und der zweiten Substanz entstehende abzugebende Substanz kann eine Flüssigkeit oder eine Emulsion sein, insbesondere eine Lösung.

Die Abgabe der abzugebenden Substanz erfolgt vorzugsweise auf automatische Art und Weise im Sinne eines Autoinjektors, und erfolgt insbesondere an einen Patienten, wie zum Beispiel den Anwender der erfindungsgemäßen Vorrichtung. Die erfindungsgemäße Vorrichtung weist dazu einen ersten Behälter auf, der eine erste Substanz enthält, und der mit einem ersten Kolben mit einer damit verbundenen sogenannten ersten Kolbenstange versehen ist, wobei der erste Behälter in der Vorrichtung beweglich angeordnet ist, sich also innerhalb der Vorrichtung in zumindest seiner Längsrichtung bzw. entlang dessen Längsachse bewegen kann. Der erste Kolben und die erste Kolbenstange sind dabei vorzugsweise einstückig ausgebildet. Die erfindungsgemäße Vorrichtung weist zudem einen zweiten Behälter auf, der eine zweite Substanz enthält, und der mit einem zweiten Kolben mit einer damit verbundenen sogenannten zweiten Kolbenstange versehen ist, wobei der zweite Behälter ebenfalls in der Vorrichtung beweglich angeordnet ist, sich also ebenfalls innerhalb der Vorrichtung in zumindest seiner Längsrichtung bzw. entlang dessen Längsachse bewegen kann. Der zweite Kolben und die zweite Kolbenstange sind vorzugsweise einstückig ausgebildet. Die vorzugsweise zylinderförmigen Behälter können innerhalb der Vorrichtung beispielsweise durch Führungsschienen oder Führungswände in der Vorrichtung in der jeweiligen Behälterlängsrichtung, also entlang der axialen Erstreckung jedes Behälters beweglich geführt sein, wobei die Vorrichtung eine maximale axiale Bewegung jedes Behälters entsprechend beschränkt. Um eine gegenseitige Beeinträchtigung der Behälter in deren Bewegung zu verhindern, können die beiden Behälter im Wesentlichen parallel, und insbesondere gleichgerichtet, zueinander in der Vorrichtung angeordnet sein. Diese Ausgestaltung der Vorrichtung erlaubt vielfältige Möglichkeiten bei der Art des zweiten Behälters und bei der Positionierung desselben relativ zum ersten Behälter, wodurch gegenüber dem bekannten Stand der Technik eine kompakte und ergonomische Vorrichtung bereitgestellt werden kann, die eine bessere Handhabbarkeit bietet und vom Patienten einfacher bei sich geführt werden kann. Bei dem ersten und dem zweiten Behälter kann es sich um eine Standardkarpule oder ein Vial handeln. Derartige Behälter sind im Stand der Technik etabliert und erfüllen insbesondere im Falle von Pharmazeutika, welche üblicherweise einem Zulassungs- und Registrierungsverfahren unterliegen, die regulatorischen Anforderungen.

Die erfindungsgemäße Vorrichtung weist weiterhin eine Injektionseinrichtung, die zum letztendlichen Injizieren der mindestens einen Substanz, d.h. des Inhalts der beiden Behälter, in den Patienten dient, und eine Leitungseinrichtung auf, die einerseits zum Leiten des Inhalts des zweiten Behälters zum bzw. in den ersten Behälter vorgesehen ist, und die andererseits ebenfalls zum Leiten des Inhalts des ersten Behälters zur bzw. in die Injektionseinrichtung vorgesehen ist. Die Injektionseinrichtung kann zur subkutanen, intramuskulären, intravenösen, intraarteriellen, intraartikulären, intrakardialen, intrakutanen, intraossären, intraperitonealen, intrapulmonalen, intrathekalen, intravitrealen oder intrazölomatischen (vorzugsweise zur subkutanen oder intramuskulären) Abgabe geeignet sein. Besonders bevorzugt ist eine subkutane Abgabe. Bei der Injektionseinrichtung kann es sich um eine einfache Injektionskanüle oder auch um eine komplexere Struktur handeln, wie sie beispielsweise aus der WO 2017/211851 A2 bekannt ist, deren Inhalt in die vorliegende Beschreibung übernommen werden kann.

Bei der erfindungsgemäßen Vorrichtung sind der Kolben des ersten Behälters, also der sogenannte erste Kolben, und der Kolben des zweiten Behälters, also der sogenannte zweite Kolben, jeweils über mindestens ein vorgespanntes Federelement nach Auslösen desselben bewegbar, das heißt dass der erste Kolben des ersten Behälters durch ein sogenanntes erstes Federelement und der zweite Kolben des zweiten Behälters durch ein sogenanntes zweites Federelement bewegt werden können, sobald das jeweilige Federelement gelöst wird und entsprechend dessen Vorspannung in eine Ausdehnung des Federelements umgesetzt wird. Dabei kann jedes der vorgespannten Federelemente in Form einer vorgespannten Spiralfeder vorliegen, deren Vorspannung sich nach deren Auslösen in eine axiale Federauslenkung des jeweiligen Federelements umwandelt. Genauer gesagt steht das erste Federelement mit dem ersten Kolben bzw. mit dem ersten Behälter direkt in Wirkverbindung, und ist über die erste Kolbenstange geführt, wobei -bei Verwendung einer Spiralfeder- beispielsweise die erste Kolbenstange innerhalb des ersten Federelements bzw. der ersten Spiralfeder angeordnet sein kann und dadurch eine Vorspannung der ersten Spiralfeder aufrechthält. Ein ähnlicher Aufbau kann bei dem zweiten Federelement zur Anwendung kommen. Ein Vorsehen eines derartigen vorgespannten Federelements bzw. derartiger vorgespannter Federelemente ermöglicht es, auf konstruktiv einfache Weise ein selbstständiges Verschieben des jeweiligen Kolbens oder des jeweiligen Behälters zu bewirken, ohne dass dazu noch ein gesonderter Antrieb erforderlich wäre.

Ein freies Ende der ersten Kolbenstange und ein freies Ende der zweiten Kolbenstange sind bei der erfindungsgemäßen Vorrichtung durch ein gemeinsames Haltebauteil lösbar gehalten, wobei das gemeinsame Haltebauteil derart gestaltet ist, dass eine Bewegung des gemeinsamen Haltebauteils zu einem nacheinanderfolgenden Lösen der ersten Kolbenstange und der zweiten Kolbenstange führt, also zuerst zu einem Lösen der ersten Kolbenstange und anschließend zu einem Lösen der zweiten Kolbenstange. Dies hat den Vorteil, dass zuerst der erste Kolben über die erste Kolbenstange und das erste Federelement in dessen Längsrichtung bzw. entlang dessen Längsachse innerhalb der Vorrichtung bewegt wird, bevor anschließend der zweite Kolben über die zweite Kolbenstange und das zweite Federelement in dessen Längsrichtung bzw. entlang dessen Längsachse innerhalb der Vorrichtung bewegt wird. Gemäß einer bevorzugten Ausführung der vorliegenden Erfindung ist dabei eine Längsachse des gemeinsamen Haltebauteils im Wesentlichen senkrecht zu den Längsachsen der ersten und der zweiten Kolbenstange angeordnet, und eine Bewegungsrichtung des gemeinsamen Haltebauteils kann im Wesentlichen senkrecht zu den Bewegungsrichtungen der beiden Kolbenstangen verlaufen. Entsprechend ist die Anordnung aus gemeinsamen Haltebauteil und den beiden Kolbenstangen in der erfindungsgemäßen Vorrichtung vorzugsweise so gestaltet, dass eine mechanische Bewegung des gemeinsamen Haltebauteils in dessen Längsrichtung zu einem nacheinanderfolgenden Lösen der ersten Kolbenstange und der zweiten Kolbenstange führt, also zuerst zu einem Lösen der ersten Kolbenstange und einer anschließenden federbedingten Bewegung der ersten Kolbenstange in deren Längsrichtung bzw. entlang deren Längsachse, das heißt im Wesentlichen senkrecht bzw. im Wesentlichen im rechten Winkel zu der Bewegung des gemeinsamen Haltebauteils, und anschließend zu einem Lösen der zweiten Kolbenstange und einer anschließenden federbedingten Bewegung der zweiten Kolbenstange in deren Längsrichtung bzw. entlang deren Längsachse, das heißt im Wesentlichen senkrecht bzw. im Wesentlichen im rechten Winkel zu der Bewegung des gemeinsamen Haltebauteils.

Aufgrund der Tatsache, dass beide Behälter wie vorhergehend ausgeführt in der Vorrichtung beweglich angeordnet sind, folgt, dass eine durch das erste Federelement ausgelöste Bewegung des ersten Kolbens eine Bewegung des ersten Behälters innerhalb der Vorrichtung hervorruft, und dass eine darauffolgende, durch das zweite Federelement ausgelöste Bewegung des zweiten Kolbens eine anschließende Bewegung des zweiten Behälters innerhalb der Vorrichtung hervorruft. Entsprechend kann über die Betätigung des gemeinsamen Haltebauteils, das auch als Triggerbauteil bzw. Auslösebauteil bezeichnet werden kann, und die daraus resultierende nacheinanderfolgende Freigabe der ersten Kolbenstange und anschließend der zweiten Kolbenstange zuerst eine der Federkraft bzw. der Federauslenkung des ersten Federelements folgende Bewegung des ersten Behälters und anschließend eine der Federkraft bzw. der Federauslenkung des zweiten Federelements folgende Bewegung des zweiten Behälters hervorgerufen werden. Das gemeinsame Haltebauteil ist damit in der Lage, zeitlich nacheinanderfolgende Bewegungen der beiden Kolben der erfindungsgemäßen Vorrichtung auszulösen, welche durch die beiden vorgespannten Federelemente verursacht werden.

Entsprechend hält das gemeinsame Haltebauteil gemäß einer bevorzugten Ausführung der vorliegenden Erfindung die erste Kolbenstange und die zweite Kolbenstange gegen die Vorspannung des jeweiligen vorgespannten Federelements zurück, hält also die erste Kolbenstange gegen die Vorspannung des ersten Federelements und die zweite Kolbenstange gegen die Vorspannung des zweiten Federelements zurück. Dabei kann das jeweilige Federelement zwischen dem gemeinsamen Haltebauteil und dem jeweiligen Kolben in Vorspannung gehalten angeordnet sein, wobei ein Freigeben des jeweiligen freien Kolbenstangenendes durch das gemeinsame Haltebauteil das Federelement freigegeben wird, also es ermöglicht wird, dass dessen Vorspannung in eine Federauslenkung umgesetzt wird, und damit die jeweilige Kolbenbewegung hervorgerufen werden kann. Gemäß einer weiteren bevorzugten Ausführung ist das gemeinsame Haltebauteil dabei im Wesentlichen plattenförmig ausgebildet, zeigt also im Wesentlichen die Gestalt einer vorzugsweise flachen Platte, die je nach Verwendung oder zusätzlichen Funktionen Aussparungen und Vorsprünge oder dergleichen haben kann, unter anderem auch fertigungsbedingter Natur. Alternativ dazu ist es ebenso denkbar, dass das gemeinsame Haltebauteil auch scheibenförmig, halbkreisförmig, oder dergleichen ausgebildet sein kann, solange eine lösbare Haltefunktion der freien Kolbenstangenenden umgesetzt werden kann.

Gemäß einer weiteren bevorzugten Ausführung der vorliegenden Erfindung weist das gemeinsame Haltebauteil zumindest eine Aussparung für das freie Ende der ersten Kolbenstange auf, also eine sogenannte erste Aussparung, und zumindest eine Aussparung für das freie Ende der zweiten Kolbenstange, also eine sogenannte zweite Aussparung. Das freie Ende der ersten Kolbenstange, das entgegengesetzt zu dem Ende der ersten Kolbenstange mit dem daran befestigten ersten Kolben angeordnet ist, das heißt von dem ersten Behälter entfernt angeordnet ist, weist dabei eine Vertiefung auf, also eine sogenannte erste Vertiefung, vorzugsweise in Form einer Nut oder dergleichen. Entsprechend dazu weist das freie Ende der zweiten Kolbenstange, das entgegengesetzt zu dem Ende der zweiten Kolbenstange mit dem daran befestigten zweiten Kolben angeordnet ist, also von dem zweiten Behälter entfernt angeordnet ist, ebenfalls eine Vertiefung auf, also eine sogenannte zweite Vertiefung, vorzugsweise in Form einer Nut oder dergleichen. Beide Vertiefungen der ersten und der zweiten Kolbenstange stehen dabei mit dem gemeinsamen Haltebauteil lösbar in Eingriff. Entsprechend überlappen sich zumindest ein Abschnitt der jeweiligen Vertiefung mit einem Abschnitt des gemeinsamen Haltebauteils, wobei dieser Haltebauteilabschnitt die jeweilige Aussparung umgibt, so dass ein mechanisches Herausziehen des entsprechenden Abschnitts des gemeinsamen Haltebauteils aus der jeweiligen Kolbenstangenvertiefung die Verbindung zwischen dem jeweiligem freien Kolbenstangenende und dem gemeinsamen Haltebauteil löst und dadurch die jeweilige Kolbenstange für dessen federgetriebene Bewegung wie vorhergehend beschrieben freigibt.

Um die vorhergehend beschriebene nacheinanderfolgende Freigabe der ersten Kolbenstange und anschließend der zweiten Kolbenstange umzusetzen, ist eine Größe des freien Endes der ersten und der zweiten Kolbenstange sowie eine Größe der jeweiligen Aussparung und/oder eine Tiefe der jeweiligen Vertiefung derart gewählt, dass die freien Enden der ersten und der zweiten Kolbenstange unterschiedlich mit dem gemeinsamen Haltebauteil zusammenwirken. Das bedeutet, dass die jeweiligen Aussparungs-Vertiefungs-Kombinationen so gewählt sind, dass die Kombination aus erster Aussparung und erster Vertiefung zu einem anderen Zeitpunkt als die Kombination aus zweiter Aussparung und zweiter Vertiefung bei einer Bewegung des gemeinsamen Haltebauteils heraus aus den Kolbenstangenenden-Vertiefungen voneinander gelöst wird. Eine zeitliche Abfolge der Freigabe der beiden freien Kolbenstangenenden kann entsprechend entweder durch die Wahl der jeweiligen Größe des jeweiligen freien Kolbenstangenendes erreicht werden, so dass unterschiedliche Überdeckungen zwischen dem jeweiligen freien Kolbenstangenende und der jeweiligen Aussparung erzielt werden, oder aber durch die Wahl unterschiedlicher Tiefen der Vertiefungen in den Kolbenstangenenden, oder aber aus einer Kombination aus diesen. Mit Überdeckung oder auch Überdeckungsbereich ist hier ein Bereich gemeint, in dem eines der freien Kolbenstangenenden durch deren Vertiefung mit dem Umfangsbereich der entsprechenden Aussparung in Eingriff steht, zu einem Zeitpunkt, zu dem das gemeinsame Haltebauteil beide Kolbenstangenenden in Eingriff hält. Entsprechend überdeckt eine Unterseite des freien Kolbenstangenendes an der dazugehörigen Vertiefung einen entsprechenden Teil des Umfangsbereichs der zugehörigen Aussparung in dem gemeinsamen Haltebauteil, so dass von einem Eingriffsbereich, einem Überdeckungsbereich oder auch einer Überdeckungsfläche gesprochen werden kann. Durch ein Herausziehen des gemeinsamen Haltebauteils aus den Vertiefungen in den Kolbenstangenenden nimmt der entsprechende Überdeckungsbereich ab, und das gemeinsame Haltebauteil geht kontinuierlich außer Eingriff, bis letztendlich kein Überdeckungsbereich verbleibt und das entsprechende freie Kolbenstangenende nicht weiter in Eingriff steht und entsprechend von dem gemeinsamen Haltebauteil losgelöst ist. Dieser Vorgang tritt bei beiden freien Kolbenstangenenden durch ein Herausziehen des gemeinsamen Haltebauteils parallel auf, wobei eine zeitliche Abfolge des Loslösens der beiden freien Kolbenstangenenden nacheinander dadurch erzielt werden kann, dass entweder die Größen der freien Kolbenstangenenden bzw. der entsprechenden Aussparungen und damit deren Überdeckungsbereiche unterschiedlich gewählt werden, oder aber durch die Wahl unterschiedlicher Tiefen der Vertiefungen in den Kolbenstangenenden, wodurch ebenfalls deren Überdeckungsbereiche unterschiedlich ausgeprägt sind, oder aber aus einer Kombination aus diesen.

In Bezug auf die vorhergehend genannten gewählten Größen der freien Kolbenstangenenden und der jeweiligen Aussparung hat dabei vorzugsweise die erste Aussparung in dem gemeinsamen Haltebauteil für das freie Ende der ersten Kolbenstange im Wesentlichen eine Dreiecksform und die zweite Aussparung in dem gemeinsamen Haltebauteil für das freie Ende der zweiten Kolbenstange im Wesentlichen eine halbrunde Form, wobei dann das freie Ende der ersten Kolbenstange ebenfalls im Wesentlichen eine Dreiecksform aufweist, und das freie Ende der zweiten Kolbenstange ebenfalls im Wesentlichen eine halbrunde Form hat. Dadurch kann neben einem erleichterten und fehlerfreieren Zusammenbauen der Vorrichtung bei der Fertigung durch eine klare formbedingte Zuweisbarkeit der jeweiligen Aussparung zu dem jeweiligen Kolbenstangenende außerdem eine unterschiedliche Überdeckung zwischen jeweiliger Vertiefung und entsprechendem Aussparungsumfang erreicht werden. Das bedeutet insbesondere, dass die Überdeckung der Vertiefung in dem halbrund geformten Kolbenstangenende und dem halbrund geformten Aussparungsumfang größer als die Überdeckung der Vertiefung in dem dreieckig geformten Kolbenstangenende und dem dreieckig geformten Aussparungsumfang ist, wobei alternative Form-Kombinationen mit derselben Wirkung ebenfalls gewählt werden können. Zusätzlich oder alternativ dazu kann die Tiefe der Vertiefung in dem freien Ende der zweiten Kolbenstange tiefer als die Tiefe der Vertiefung in dem freien Ende der ersten Kolbenstange sein, wodurch ebenfalls eine zeitliche Abfolge des Freigebens des freien Endes der ersten Kolbenstange und anschließend des Freigebens des freien Endes der zweiten Kolbenstange erreicht wird. Ferner kann das freie Ende der zweiten Kolbenstange aufgrund dessen halbrunder und damit drehbarer Querschnittsgestalt einen Vorsprung, beispielsweise in Form eines Stegs oder einer Nase, als Verdrehsicherung aufweisen, um in Verbindung mit einer entsprechenden Vertiefung als Gegenstück ein Verdrehen des freien Endes der zweiten Kolbenstange zu verhindern. Demgegenüber kann auf einen entsprechenden Vorsprung bei dem freien Ende der ersten Kolbenstange verzichtet werden, da dieses durch dessen dreieckige Form und dem entsprechenden Gegenstück bereits gegen ein Verdrehen weitestgehend abgesichert ist.

Gemäß einer weiteren bevorzugten Ausführung der vorliegenden Erfindung bewegt das mindestens eine vorgespannte Federelement des ersten Kolbens, d.h. das erste Federelement, nach einem Lösen der ersten Kolbenstange durch das gemeinsame Haltebauteil den ersten Kolben zusammen mit dem ersten Behälter gegenüber der Vorrichtung. Ferner bewegt das mindestens eine vorgespannte Federelement des zweiten Kolbens, d.h. das zweite Federelement, nach einem Lösen der zweiten Kolbenstange zunächst den zweiten Kolben zusammen mit dem zweiten Behälter gegenüber der Vorrichtung und darauffolgend den zweiten Kolben gegenüber dem zweiten Behälter. Gemäß einer diesbezüglich weiteren bevorzugten Ausführung der vorliegenden Erfindung ist die erste Substanz, die in dem ersten Behälter vorliegt, von der in dem zweiten Behälter vorliegenden zweiten Substanz verschieden. Entsprechend führt ein Mischen der ersten mit der zweiten Substanz zu einer Substanzmischung, die aus den beiden Substanzen besteht, wie bereits an vorhergehender Stelle ausführlich beschrieben. Unter der Voraussetzung dass die beiden Behälter miteinander in Fluidverbindung gebracht sind, wird durch Verschieben des zweiten Kolbens gegen das entsprechende Behälterende des zweiten Behälters die als Flüssigkeit vorliegende zweite Substanz aus dem zweiten Behälter herausgedrückt und in den ersten Behälter übertragen, in dem sich die Flüssigkeit mit der darin vorliegenden ersten Substanz mischt und diese eine entsprechende Lösung ausbilden können. Anschließend kann dann die daraus resultierende Lösung aus erster Substanz und Lösungsflüssigkeit in dem ersten Behälter durch Verschieben des ersten Kolbens gegen das entsprechende Behälterende des ersten Behälters über die Injektionseinrichtung, die mit dem ersten Behälter in Fluidverbindung steht, abgegeben werden.

Dabei kann die erste Substanz ein Feststoff sein, wie zum Beispiel ein Lyophilisat, so dass ein Mischen von Lösungsflüssigkeit und Lyophilisat zu einer Rekonstitution des Lyophilisats führt, so dass dieses im Anschluss aus der Vorrichtung und über die Injektionseinrichtung in den Patienten eingespritzt werden kann. Bei einer derartigen Anwendung kann der erste Behälter auch als Lyophilisatbehälter bezeichnet werden, und der zweite Behälter kann als Flüssigkeitsbehälter bezeichnet werden. Eine Rekonstitution des Lyophilisats ist hierbei unabhängig von der Orientierung der Vorrichtung relativ zur Schwerkraft möglich. Generell kann im Hinblick auf die vorliegend beschriebenen Kolbenbewegungen gemäß einer weiteren bevorzugten Ausführung der vorliegenden Erfindung der erste Behälter ein erstes Behälterende und ein zweites Behälterende aufweisen, und der zweite Behälter kann ein entsprechendes erstes und zweites Behälterende aufweisen, wobei der erste und der zweite Kolben zwischen dem jeweiligen ersten Behälterende und dem jeweiligen zweiten Behälterende axial verschiebbar sind.

Entsprechend kann durch Verschieben des ersten Kolbens gegen das erste Behälterende des ersten Behälters ein Inhalt des ersten Behälters aus dem ersten Behälter abgegeben werden, und durch Verschieben des zweiten Kolbens gegen das erste Behälterende des zweiten Behälters ein Inhalt des zweiten Behälters aus dem zweiten Behälter abgegeben werden, wobei insbesondere bei Vorliegen einer Fluidverbindung zwischen Injektionseinrichtung und erstem Behälter durch das Verschieben des ersten Kolbens gegen das erste Behälterende des ersten Behälters ein Inhalt des ersten Behälters über die Injektionseinrichtung nach außen abgebbar ist.

Gemäß einer weiteren bevorzugten Ausführung der vorliegenden Erfindung kann die Leitungseinrichtung ein erstes Anschlusselement für eine Fluidverbindung mit dem ersten Behälter und ein zweites Anschlusselement für eine Fluidverbindung mit dem zweiten Behälter aufweisen, wobei jedes Anschlusselement als ein nadelartiger Vorsprung zum Durchdringen eines Septums des jeweiligen Behälters ausgeführt sein kann. Ein Septum ermöglicht ein besonders zuverlässiges und dichtes Abschließen eines Behälters. In beiden genannten Fällen kann der nadelartige Vorsprung in Form einer Nadel, insbesondere einer schräg angeschliffenen Hohlkanüle, vorliegen. Um das Risiko einer Kontamination der abzugebenden Substanz zu verringern, ist es insbesondere bei Sterilanwendungen vorteilhaft, wenn verwendete Penetrationsmittel wie die genannten Nadeln bzw. Hohlkanülen jeweils mit einer flexiblen Schutzkappe oder Schutzhülse versehen sind, die ebenfalls zusammen mit dem entsprechenden Septum durchstoßen werden können. Der Einsatz derartiger Schutzkappen oder Schutzhülsen erlaubt insbesondere eine Endmontage der Vorrichtung unter nicht sterilen Bedingungen. Ferner kann die Leitungseinrichtung eine Fluidverbindung zu der Injektionseinrichtung aufweisen, wobei in der Leitungseinrichtung ein Ventilelement in Form eines Einwegventils vorgesehen ist, so zum Beispiel ein Rückschlagventil, beispielsweise in Gestalt eines Klappventils, das insbesondere in der Fluidverbindung zu der Injektionseinrichtung als Einwegventil wirkt. Das Ventilelement ist dabei so in der Leitungseinrichtung vorgesehen, dass eine Flüssigkeitsübertragung von dem zweiten Behälter in den ersten Behälter möglich ist, eine anschließende Flüssigkeitsübertragung von dem ersten Behälter in den zweiten Behälter verhindert wird, aber eine Flüssigkeitsübertragung von dem ersten Behälter an die Injektionsvorrichtung erlaubt ist. Dabei können vorzugsweise der Anschluss für den ersten Behälter und der Anschluss für die Injektionseinrichtung auf derselben Seite des Einwegventils liegen, wohingegen der Anschluss für den zweiten Behälter auf der anderen Seite des Einwegventils liegt. Gemäß einer weiteren bevorzugten Ausführung der vorliegenden Erfindung unter diesem Aspekt stellt das Lösen des freien Endes der ersten Kolbenstange und die damit ausgelöste Bewegung des ersten Kolbens durch das erste Federelement eine Fluidverbindung zwischen der Leitungseinrichtung und dem ersten Behälter her, beispielsweise durch Anstechen bzw. Andocken des ersten Behälters mittels des zur Leitungseinrichtung gehörenden nadelartigen Vorsprungs zum Durchdringen des Septums des ersten Behälters. Ferner stellt ein darauffolgendes Lösen des freien Endes der zweiten Kolbenstange und die damit ausgelöste Bewegung des zweiten Kolbens durch das zweite Federelement eine Fluidverbindung zwischen der Leitungseinrichtung und dem zweiten Behälter her, beispielsweise ebenfalls durch Anstechen bzw. Andocken des zweiten Behälters mittels des zur Leitungseinrichtung gehörenden nadelartigen Vorsprungs zum Durchdringen des Septums des zweiten Behälters. Beide nadelartigen Vorsprünge können dabei in Form einer Nadel vorliegen, die an der Leitungseinrichtung mit dieser in Fluidverbindung stehend vorgesehen sind und in Richtung des jeweiligen Behälters bzw. des jeweiligen ersten Behälterendes ausgerichtet sind, an dem sich jeweils ein Septum oder dergleichen befinden kann, um eine Fluidverbindung zwischen Nadel und Behälterinhalt zu ermöglichen. Zusätzlich dazu bewirkt das zweite Federelement, welches vorzugsweise eine baulich bedingte größere Federauslenkung als das erste Federelement hat, zudem, dass nicht nur der zweite Behälter zusammen mit dem zweiten Kolben gegen den entsprechenden nadelartigen Vorsprung gedrückt wird, sondern ferner dass nach einem Andocken des zweiten Behälters an dem nadelartigen Vorsprung und damit einem Ende der axialen Bewegung des zweiten Behälters der zweite Kolben durch das zweite Federelement weiter bewegt und in dem zweiten Behälter gegen das Behälterende des zweiten Behälters gedrückt wird, wodurch die zweite Substanz aus dem zweiten Behälter über die Leitungseinrichtung in den ersten Behälter übertragen wird. Durch das hiermit erreichte zeitversetzte Andocken der beiden Behälter an die Leitungseinrichtung kann sichergestellt werden, dass die Flüssigkeit aus dem zweiten Behälter erst in die Leitungseinrichtung abgegeben wird, wenn der erste Behälter bereits angedockt ist.

Für das weitere Verschieben des ersten Kolbens gegen das entsprechende erste Behälterende des ersten Behälters, um den Inhalt des ersten Behälters an die Injektionseinrichtung zu übertragen und durch diese abgegeben zu werden, ist bei der erfinderischen Vorrichtung eine zusätzliche Bewegungseinrichtung notwendig, die beispielsweise in Form eines zusätzlichen vorgespannten Federelements vorgesehen sein kann, welches neben dem ersten Federelement ebenfalls an dem ersten Kolben angebracht ist und mit diesem in Wirkverbindung steht. Eine anschließende Aktivierung des zusätzlichen Federelements führt entsprechend dazu, dass bei Vorliegen einer Fluidverbindung zwischen Injektionseinrichtung und erstem Behälter durch das Verschieben des ersten Kolbens gegen das erste Behälterende des ersten Behälters ein Inhalt des ersten Behälters über die Injektionseinrichtung nach außen abgegeben werden kann.

Gemäß einer weiteren bevorzugten Ausführung der vorliegenden Erfindung kann die Vorrichtung ferner ein Aktivierungsbauteil aufweisen, das mit dem gemeinsamen Haltebauteil in mechanischer Wirkverbindung steht. Das bedeutet, dass das Aktivierungsbauteil und das gemeinsame Haltebauteil miteinander mechanisch interagieren können, wobei insbesondere eine Bewegung des Aktivierungsbauteils zu der vorhergehend bereits beschriebenen Bewegung des gemeinsamen Haltebauteils in dessen Längsrichtung führt. Entsprechend kann die Bewegung des gemeinsamen Haltebauteils in dessen Längsrichtung, die das nacheinanderfolgende Lösen der ersten Kolbenstange und der zweiten Kolbenstange auslöst, durch eine Bewegung des Aktivierungsbauteils hervorgerufen werden. Dabei ist es eine weiter bevorzugte Ausführung der vorliegenden Erfindung, dass eine Längsachse des Aktivierungsbauteils im Wesentlichen senkrecht zu der Längsachse des gemeinsamen Haltebauteils angeordnet ist, also im Wesentlichen parallel zu den Längsachsen der beiden Kolbenstangen und damit der beiden Behälter, wobei eine Bewegungsrichtung des Aktivierungsbauteils in dessen Längsrichtung vorzugsweise im Wesentlichen senkrecht bzw. im Wesentlichen im rechten Winkel zu der Bewegungsrichtung des gemeinsamen Haltebauteils in dessen Längsrichtung verläuft. Vorzugsweise kann das Aktivierungsbauteil eine Kaskadenfläche aufweisen, und das gemeinsame Haltebauteil kann eine Aktivierungsaussparung aufweisen, wobei das Aktivierungsbauteil mit der Kaskadenfläche in der Aktivierungsaussparung angeordnet ist, und ein Herausziehen des Aktivierungsbauteils aus der Aktivierungsaussparung eine axiale Bewegung des gemeinsamen Haltebauteils hervorruft. Beispielsweise kann hier das Herausziehen des Aktivierungsbauteils durch ein Entfernen einer Abdeckung der erfindungsgemäßen Vorrichtung erreicht werden; insbesondere kann das Aktivierungselement mit der Abdeckung verbunden sein und somit durch ein Auspacken und Aktivieren der Vorrichtung beim Auspacken der Vorrichtung entfernt werden, wodurch zur Rekonstitution des Lyophilisats in der Vorrichtung kein weiterer separater Schritt und keine weitere separate Handlung durch den Anwender erforderlich sind. Alternativ dazu kann die vorhergehend beschriebene Aktivierung durch Bewegung des gemeinsamen Haltebauteils in dessen Längsrichtung, um das nacheinanderfolgende Lösen der ersten Kolbenstange und der zweiten Kolbenstange auszulösen, durch einen alternativen Wirkmechanismus erzielt werden, welche beispielsweise aus einem mit dem gemeinsamen Haltebauteil verbundenen, stiftförmigen Betätigungselement und einer entsprechenden Steuerungsnut in der Abdeckung erzielt werden, wie es weiter unten der Figurenbeschreibung genauer entnommen werden kann.

Gemäß einem weiteren Gesichtspunkt der vorliegenden Erfindung wird zudem ein Verfahren zur Bereitstellung einer gemischten Substanz, bereitgestellt, wobei für das erfindungsgemäße Verfahren eine Vorrichtung wie vorhergehend beschrieben zur Anwendung kommt. In einem weiteren nicht erfindungsgemäßen Verfahren kann es sich um eine Autoinjektion durch die vorhergehend beschriebene Vorrichtung handeln, und die bereits für die Vorrichtung vorhergehend beschriebenen Merkmale und Abläufe sind entsprechend ebenfalls für das erfindungsgemäße Verfahren anwendbar.

Das erfindungsgemäße Verfahren umfasst insbesondere einen Schritt des Bewegens des mit dem gemeinsamen Haltebauteil in Wirkverbindung stehenden Aktivierungsbauteils entlang dessen Längsachse, wodurch das gemeinsame Haltebauteil im Wesentlichen in dessen Längsrichtung bewegt, insbesondere senkrecht zu der Bewegungsrichtung des Aktivierungsbauteils, und dabei zuerst den Eingriff zwischen dem gemeinsamen Haltebauteil und dem freien Ende der ersten Kolbenstange und darauffolgend den Eingriff zwischen dem gemeinsamen Haltebauteil und dem freien Ende der zweiten Kolbenstange löst. Entsprechend kann die Bewegung des gemeinsamen Haltebauteils in dessen Längsrichtung, die das nacheinanderfolgende Lösen der ersten Kolbenstange und der zweiten Kolbenstange auslöst, durch eine Bewegung des Aktivierungsbauteils in dessen Längsrichtung bzw. entlang dessen Längsachse hervorgerufen werden. Dabei kann das Lösen des freien Endes der ersten Kolbenstange aufgrund des entsprechenden ersten Federelements eine Fluidverbindung zwischen der Leitungseinrichtung und dem ersten Behälter bewirken, und das darauffolgende Lösen des freien Endes der zweiten Kolbenstange aufgrund des entsprechenden zweiten Federelements kann eine Fluidverbindung zwischen der Leitungseinrichtung und dem zweiten Behälter bewirken, wobei durch die Federauslenkung des zweiten Federelements anschließend zudem der Inhalt des zweiten Behälters über die Leitungseinrichtung in den ersten Behälter übertragen wird. Ferner kann das Verfahren einen zusätzlichen Schritt des Verschiebens des ersten Kolbens gegen das erste Behälterende des ersten Behälters umfassen, wodurch der Inhalt des ersten Behälters über die Injektionseinrichtung abgeben wird. Dies hat den Vorteil, dass nach dem Mischen der Inhalte der beiden Behälter durch eine einzelne Aktion des Anwenders, und zwar durch die Bewegung des Aktivierungsbauteils, und nach dem Anbringen der Vorrichtung am Körper des Anwenders nur noch eine zweite Betätigungshandlung des Anwenders im Sinne des zusätzlichen Schrittes zum Auslösen der Injektion notwendig ist. Insbesondere kann der Injektionsauslöseschritt im Drücken einer Taste bestehen. Dadurch können in einer vordefinierten zeitlichen Abfolge mechanische Kräfte auf Bestandteile der Vorrichtung übertragen werden. Die vorliegende Erfindung kommt also mit einem Minimum an Handlungen seitens des Anwenders aus. Die Bedienung der Vorrichtung wird dadurch einfacher, angenehmer und weniger fehleranfällig. Einzelheiten zu den jeweiligen Vorgängen sind bereits in Verbindung mit der vorhergehenden Beschreibung der erfindungsgemäßen Vorrichtung beschrieben und werden daher an dieser Stelle nicht wiederholt.

Wie hier und auch in den beigefügten Ansprüchen eventuell verwendet können die Singularformen "ein"/"eine"/"einer" und "der"/"die"/"das" auch deren Plural umfassen, sofern der Kontext nicht eindeutig etwas anderes vorgibt. In ähnlicher Weise sind die Wörter "umfassen", "enthalten" und "aufweisen" sowohl als "ausschließlich" als auch "nicht ausschließlich" zu verstehen, also im Sinne von "einschließlich, aber nicht beschränkt auf...". Die Begriffe "mehrere", "Vielfaches" oder "Vielzahl" beziehen sich üblicherweise auf zwei oder mehr, d.h. 2 oder >2, einschließlich weiterer ganzzahliger Vielfacher von 1, wobei sich die Begriffe "einzeln" oder "allein" auf eins (1) beziehen, also "=1". Ferner ist der Ausdruck "mindestens eins" oder "wenigstens eins" als eins oder mehrere, d.h. 1 oder >1, ebenfalls mit ganzzahligen Vielfachen, zu verstehen. Wenn das Wort "zwischen" verwendet wird, um einen numerischen Bereichen zu beschreiben, sollen die Randpunkte des genannten Bereichs explizit als Teil des Bereichs gelten. Außerdem sollen sich die Wörter "hierin", "oben", "vorher" und "unten" oder "nachfolgend" und Wörter mit ähnlicher Bedeutung, wenn sie in dieser Beschreibung verwendet werden, auf diese Beschreibung insgesamt und nicht auf bestimmte Teile der Beschreibung beziehen.

Die nachfolgenden Ausführungen zu spezifischen Ausführungsformen in dieser Beschreibung sollen nicht als erschöpfend angesehen werden, oder die hierin gegebene Offenbarung soll nicht auf die genaue offenbarte Form beschränkt werden. Während hierin beschriebene spezifische Ausführungsformen und Beispiele für die Offenbarung zur Veranschaulichung dienen, sind verschiedene äquivalente Modifikationen innerhalb des Schutzumfangs der Offenbarung möglich, wie es von einem Fachmann auf dem vorliegenden technischen Gebiet erkennbar ist. Spezielle technische Elemente von beschriebenen Ausführungsformen können mit technischen Elementen in anderen Ausführungsformen kombiniert oder durch diese ersetzt werden. In den Zeichnungen bezeichnen gleiche Bezugszeichen gleiche Elemente um Wiederholungen zu vermeiden, und Teile, die der Fachmann ohne spezielles Wissen umsetzen kann, können aus Gründen der Übersichtlichkeit weggelassen werden. Während Vorteile, die bestimmten Ausführungsformen der Offenbarung zugeordnet sind, im Zusammenhang mit diesen Ausführungsformen beschrieben werden, können andere Ausführungsformen ebenfalls diese Vorteile aufweisen, ohne selbe explizit anzuführen.

Die nachfolgenden Beispiele sollen spezifische Ausführungsformen der vorliegenden Erfindung anhand der Figuren veranschaulichen. Alle spezifischen Modifikationen, wie sie nachfolgend ebenfalls diskutiert werden, sind als solche nicht als Beschränkungen des Umfangs der vorliegenden Erfindung auszulegen. Für den Fachmann ist es offensichtlich, dass verschiedene Abwandlungen, Änderungen und Modifikationen vorgenommen werden können, ohne von dem Schutzbereich der vorliegenden Offenbarung abzuweichen, wie er durch die angehängten Ansprüche festgelegt ist. Weitere Aspekte und Vorteile der vorliegenden Erfindung ergeben sich aus der folgenden Beschreibung der bevorzugten, in den Figuren dargestellten Ausführungsformen.

### FIGURENBESCHREIBUNG

Es zeigen:
- Figur 1: eine perspektivische schematische Ansicht einer erfindungsgemäßen Vorrichtung nach einer bevorzugten Ausführungsform in explodierter Darstellung;
- Figur 2: eine perspektivische schematische Ansicht eines Hauptkörpers der in Figur 1 gezeigten Vorrichtung in einer längsgeschnittenen Darstellung;
- Figur 3: eine Draufsicht der in Figur 2 dargestellten Ansicht;
- Figur 4: eine Draufsicht eines Teils der in Figur 2 gezeigten Ansicht in explodierter Darstellung, die insbesondere ein Zusammenwirken zwischen dem gemeinsamen Haltebauteil und den beiden Kolbenstangen zeigt;
- Figur 5: eine perspektivische Ansicht der in Figur 4 gezeigten Darstellung;
- Figuren 6a bis 6c: einen Loslösevorgang der beiden Kolbenstangenenden durch eine Bewegung des gemeinsamen Haltebauteils in drei exemplarischen Schritten;
- Figur 7: ein Aktivieren des gemeinsamen Haltebauteils der in den vorherigen Figuren gezeigten Vorrichtung der bevorzugten Ausführungsform durch ein Aktivierungsbauteil;
- Figuren 8a bis 8c: einen Loslösevorgang der beiden Kolbenstangenenden durch eine Bewegung einer alternativen Ausführungsform des gemeinsamen Haltebauteils in drei exemplarischen Schritten, ausgelöst durch einen alternativen Wirkmechanismus;
- Figur 9a: eine perspektivische schematische Ansicht einer alternativen Ausführungsform einer Abdeckung der erfindungsgemäßen Vorrichtung; und
- Figur 9b: eine perspektivische schematische Ansicht einer Innenseite einer unteren Hälfte der in Fig. 9a gezeigten Abdeckung, dargestellt in einer Draufsicht auf eine Schnittebene, welche entlang der Schnittlinie B-B verläuft.

### BEVORZUGTE AUSFÜHRUNGSFORMEN DER ERFINDUNG

In der nachfolgenden Beschreibung der bevorzugten Ausführungsformen der Erfindung stellen die Figuren den Gegenstand der Erfindung nur schematisch dar. Die bevorzugten Ausführungsformen der Erfindung sind in den Zeichnungen dargestellt und werden im Folgenden näher beschrieben.

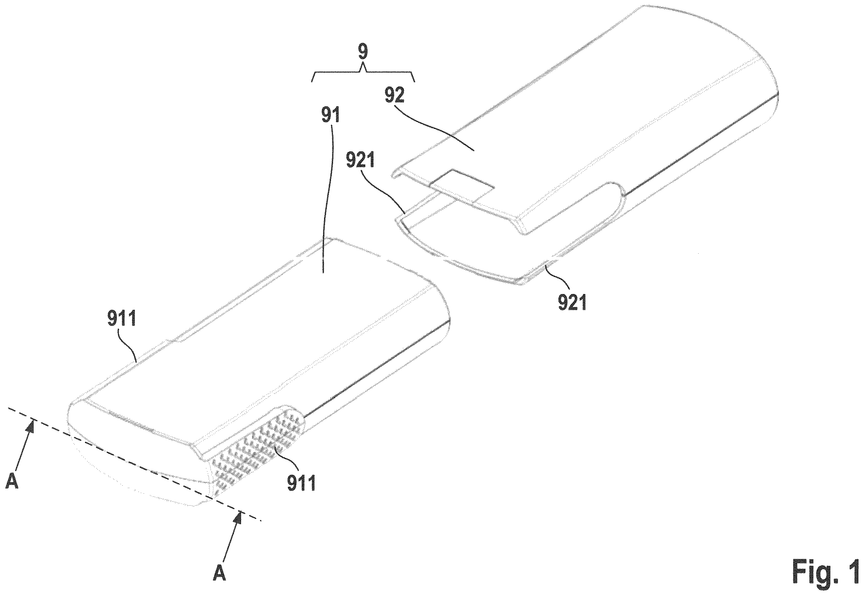

Figur 1 zeigt dabei eine perspektivische schematische Ansicht einer erfindungsgemäßen Vorrichtung 9 nach einer bevorzugten Ausführungsform der Erfindung in explodierter Darstellung. Dabei ist die Vorrichtung 9 in zwei auseinandergebauten Hauptbestandteilen dargestellt, und zwar einen Hauptkörper 91 der Vorrichtung 9 sowie eine Abdeckung 92, welche im zusammengebauten Zustand der Vorrichtung 9 den Hauptkörper 91 zumindest teilweise abdeckt. Der Hauptkörper 91 weist in der hier dargestellten bevorzugten Ausführungsform eine im Wesentlichen rechteckige Gestalt mit abgerundeten Kanten auf. Die Abdeckung 92 zeigt eine der Gestalt des einzuschiebenden Hauptkörpers 91 entsprechende hohle Gestalt mit einer offenen Seite, wobei an der offenen Seite der Abdeckung beiderseits Aussparungen 921 vorgesehen sind. An entsprechender Stelle an dem Hauptkörper 91 vorgesehene Griffleisten 911 werden im zusammengebauten Zustand in diese Aussparungen 921 eingeschoben, um ein Herausziehen des Hauptkörpers 91 aus der Abdeckung 92 bzw. ein Abnehmen der Abdeckung 92 von dem Hauptkörper 91 durch einen Anwender zu erleichtern.

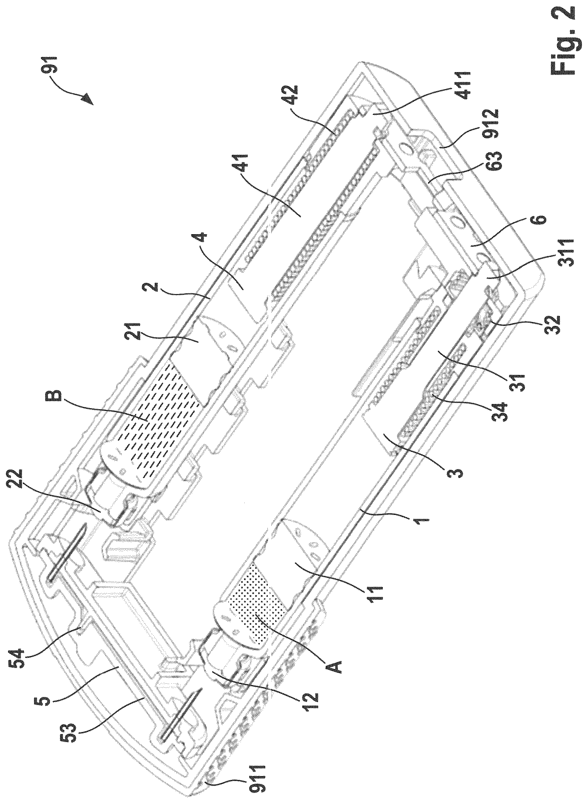

Der in Figur 1 dargestellte Hauptkörper 91 ist in Figur 2 in einer entlang der Linie A-A entlang dessen Längsachse geschnittenen Darstellung gezeigt. Darin ist zu erkennen, dass der Hauptkörper 91 an dessen einem Ende, welches im zusammengebauten Zustand in die Abdeckung 92 eingeschoben sein soll, eine Öffnung 912 aufweist. Zudem ist an dem Hauptkörper 91 eine weitere, durch eine Versiegelung abgedeckte Öffnung (nicht gezeigt) bereitgestellt, beispielsweise an der Unterseite des Hauptkörpers 91, deren Funktion an späterer Stelle erklärt wird. In dem ansonsten im Wesentlichen geschlossenen Hauptkörper 91 befindet sich unter anderem ein erster Behälter 1, in dem eine erste Substanz A durch einen Stopfen 11 verschlossen aufgenommen ist, wobei die Substanz A bei der vorliegenden Ausführungsform in Gestalt eines Lyophilisats vorliegt. Das andere Ende des ersten Behälters 1, d.h. das zu dem Stopfen 11 entgegengesetzte Ende des ersten Behälters 1, das auch als erstes Ende des ersten Behälters 1 bezeichnet werden kann, ist beispielsweise durch ein Septum 12 verschlossen. Der erste Behälter 1, der in diesem Sinne auch als Lyophilisatbehälter 1 bezeichnet werden kann, ist in dem vorliegenden Fall auf der linken Seite in Fig. 2 angeordnet. In dem Hauptkörper 91 befindet sich ferner ein zweiter Behälter 2, in dem eine zweite Substanz B durch einen Stopfen 21 verschlossen aufgenommen ist, wobei die Substanz B bei der vorliegenden Ausführungsform in Gestalt einer Lösungsflüssigkeit zur Rekonstitution des Lyophilisats vorliegt. Das andere Ende des zweiten Behälters 2, d.h. das zu dem Stopfen 21 entgegengesetzte Ende des zweiten Behälters 2, das auch als erstes Ende des zweiten Behälters 2 bezeichnet werden kann, ist beispielsweise durch ein Septum 22 verschlossen. Der zweite Behälter 2, der in diesem Sinne auch als Lösungsflüssigkeitsbehälter 2 bezeichnet werden kann, ist in dem vorliegenden Fall auf der rechten Seite in Fig. 2 angeordnet. Beide Behälter 1 und 2 sind auswechselbar in dem Hauptkörper 91 angeordnet.

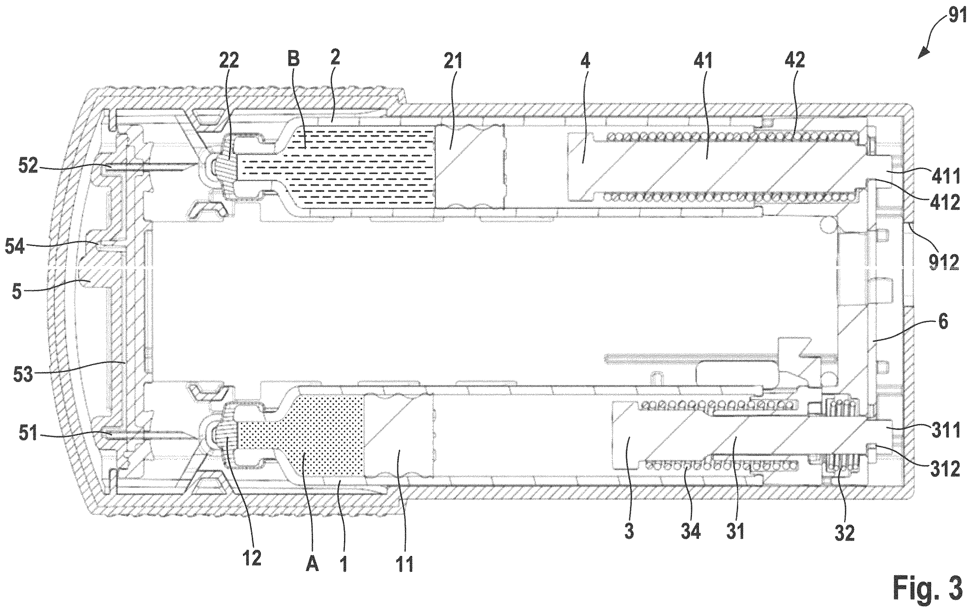

Wie es weiterhin sowohl in Figur 2 als auch in der dazu entsprechenden Draufsicht in Figur 3 zu sehen ist, ist in dem Hauptkörper 91 zudem ein erster Kolben 3 mit einer ersten Kolbenstange 31 angeordnet, wobei der erste Kolben 3 bzw. dessen Kolbendach im Wesentlichen innerhalb des Behälters 1 so geführt ist, dass das Kolbendach mit der gegenüberliegenden Seite des Stopfens 11 in Kontakt treten kann, so dass der Stopfen 11 gegen das Septum-tragende Ende des ersten Behälters 1, welches ein erstes Ende des ersten Behälters 1 darstellt, gedrückt werden kann. Ferner ist in dem Hauptkörper 91 ein zweiter Kolben 4 mit einer sogenannten zweiten Kolbenstange 41 angeordnet, wobei der zweite Kolben 4 bzw. dessen Kolbendach im Wesentlichen innerhalb des zweiten Behälters 2 so geführt ist, dass das Kolbendach mit der gegenüberliegenden Seite des Stopfens 21 in Kontakt treten kann, so dass der Stopfen 21 gegen das Septum-tragende Ende des zweiten Behälters 2, welches ein erstes Ende des zweiten Behälters 2 darstellt, gedrückt werden kann. Beide Behälter 1 und 2 sind derart in dem Hauptkörper 91 montiert, dass diese in deren Längsrichtung in dem Hauptkörper 91 bewegbar gelagert oder geführt sind, wie es in Figuren 2 und 3 angedeutet ist. Bei einer Bewegung der Behälter 1 und 2 in Richtung deren Septum-tragender Enden wird das jeweilige Septum 12, 22 auf eine jeweilige fest angeordnete Nadel 51, 52 gedrückt, wodurch die Septen 12, 22 von den Nadeln 51, 52 durchstochen werden. Durch dieses Anstechen der Septen 12, 22, und damit das Anstechen der Behälter 1, 2, wird eine Fluidverbindung zwischen dem Inhalt der Behälter 1, 2 und einem Leitungskanal 53 einer Leitungseinrichtung 5 hergestellt, die an einem in den Figuren 2 und 3 dargestellten linken Ende an der Innenseite des Hauptkörpers 91 fest angeordnet ist. Die Leitungseinrichtung 5, die fertigungsbedingt aus mehreren Wandbauteilen bestehen kann, wie in den Figuren 2 und 3 angedeutet, hält nicht nur die Nadel 51 und die Nadel 52 an Ort und Stelle, ausgerichtet auf das jeweilige Septum 12, 22, sondern bildet in dessen Inneren den Leitungskanal 53 aus. In dem Leitungskanal 53 ist auch ein Einwegventil 54 vorgesehen, hier in Form einer Gummilippe, die wie eine Klappe auf einer Seite gegen eine Innenwand des Leitungskanals 53 anliegt und damit in der vorliegenden Ausführungsform einen Fluidfluss von dem zweiten Behälter 2 in den ersten Behälter 1 zulässt, jedoch einen Rückfluss eines Fluids von dem ersten Behälter 1 in den zweiten Behälter 2 verhindert. Neben den bereits beschriebenen Bauteilen ist in dem Leitungskanal 53 ferner eine vorzugsweise versiegelte Öffnung (nicht gezeigt) zum Anschluss einer Injektionseinrichtung (nicht gezeigt) vorgesehen, und zwar in dem Bereich des Leitungskanals 53, der ausgehend von dem Einwegventil 54 auf der Seite des ersten Behälters 1 liegt. Die (nicht gezeigte) Injektionseinrichtung kann dabei so ausgebildet sein, dass diese ebenfalls durch eine Nadel oder dergleichen die versiegelte Öffnung anstechen kann, oder dass die Injektionseinrichtung bereits mit der versiegelten Öffnung verbunden ist. Die Injektionseinrichtung dient dazu, eine sich in dem Leitungskanal 53 und/oder in dem Behälter 1 befindliche Substanz an einen Anwender durch Injektion, vorzugsweise Autoinjektion, abzugeben. Dazu kommt die bereits vorhergehend erwähnte, durch eine Versiegelung abgedeckte Öffnung (nicht gezeigt) zur Anwendung, die beispielsweise an der Unterseite des Hauptkörpers 91 vorgesehen ist, und durch die die Injektionseinrichtung hindurchstechend die abzugebende Substanz ausstoßen kann.

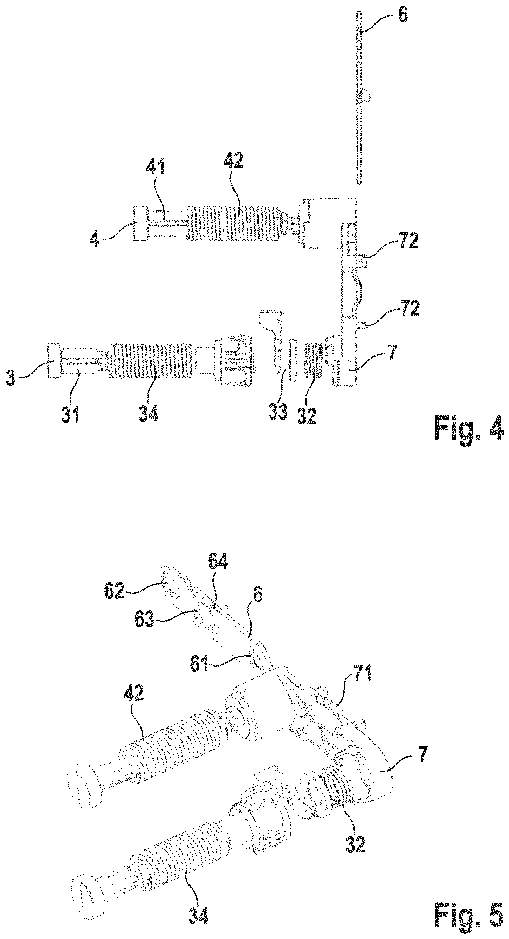

Das oben beschriebene Anstechen der Behälter 1, 2 durch das Durchstechen der Septen 12, 22 mittels der Nadel 51, 52 wird wie besprochen durch ein Bewegen der Behälter 1, 2 in deren axialer Richtung bewirkt. Um eine derartige Bewegung des ersten Behälters 1 hervorzurufen ist der erste Behälter 1 in der gezeigten bevorzugten Ausführungsform mit einem ersten vorgespannten Federelement 32 versehen, hier in Gestalt einer Spiralfeder 32, welche an einem dem ersten Behälter 1 abgewandten Ende durch Anlage an ein Führungsbauteil 7 gehalten ist und an einem dem ersten Behälter 1 zugewandten Ende über ein Zwischenbauteil 33 mit dem ersten Behälter 1 in Wirkverbindung steht, so dass eine Auslenkung der vorgespannten Spiralfeder 32 eine Längsverschiebung des ersten Behälters 1 in Richtung hin zu der Nadel 51 bewirkt, um das Septum 12 des ersten Behälters 1 mit der Nadel 51 anzustechen. Die Spiralfeder 32 befindet sich durch eine Klemmung zwischen dem Zwischenbauteil 33 und dem Führungsbauteil 7 in Vorspannung, wobei die Spiralfeder 32 um die erste Kolbenstange 31 herum angeordnet ist und ein freies Ende 311 der ersten Kolbenstange 31 des ersten Kolbens 3 durch ein Haltebauteil 6 an Ort und Stelle gehalten wird. Das Haltebauteil 6 wird auch als gemeinsames Haltebauteil 6 bezeichnet, da es nicht nur das freie Ende 311 der ersten Kolbenstange 31 des ersten Kolbens 3, sondern zudem das freie Ende 411 der zweiten Kolbenstange 41 des zweiten Kolbens 4 durch Eingriff mit diesen an Ort und Stelle hält. An der Kolbenkonstruktion des ersten Kolbens 3 des ersten Behälters 1 ist in der vorliegenden beschriebenen Ausführungsform zudem ein weiteres vorgespanntes Federelement 34 in Gestalt einer Spiralfeder 34 angeordnet, welches nach einem Auslösen beispielsweise durch einen separaten, von dem Anwender zu betätigenden Mechanismus gelöst werden kann, so dass eine Auslenkung der weiteren vorgespannten Spiralfeder 34 bewirkt wird, welche eine Bewegung des ersten Kolbens 3, geführt über die erste Kolbenstange 31, in Richtung hin zu der bereits durch das erste vorgespannte Federelement 32 hergestellten Fluidverbindung zwischen dem ersten Behälter 1 und dem Leitungskanal 53 bewirkt, so dass ein Inhalt des ersten Behälters 1 in den Leitungskanal 53 und darüber hinaus durch die Injektionseinrichtung (nicht gezeigt) hindurch und aus dem Hauptkörper 91 der Vorrichtung 9 hinaus gedrückt werden kann. Ein genauer Aufbau des Zusammenspiels von Kolben 3, Kolbenstange 31, erstem vorgespannten Federelement 32, Zwischenbauteil 33 und weiterem vorgespannten Federelement 34 kann ebenfalls den Figuren 4 und 5 entnommen werden, in denen das generelle Zusammenwirken zwischen den beiden Kolbenstrukturen der Kolben 3 und 4 sowie deren Federelementen 32, 34 und 42 sowie dem gemeinsamen Haltebauteil 6 freigeschnitten von den restlichen Bauteilen der Vorrichtung 9 zur vereinfachten Darstellung in einer Explosionsansicht perspektivisch sowie in Draufsicht gezeigt ist.

Um entsprechend eine Anstechbewegung des zweiten Behälters 2 hervorzurufen, ist der zweite Behälter 2 in der gezeigten bevorzugten Ausführungsform mit einem zweiten vorgespannten Federelement 42 versehen, hier in Gestalt einer Spiralfeder 42, welche an einem dem zweiten Behälter 2 abgewandten Ende durch Anlage an das Führungsbauteil 7 gehalten ist und an einem dem zweiten Behälter 2 zugewandten Ende durch den zweiten Kolben 4 mit dem zweiten Behälter 2 in Wirkverbindung steht, so dass eine Auslenkung der vorgespannten Spiralfeder 42 eine Längsverschiebung des zweiten Kolbens 4 gegen den Stopfen 21 und dadurch eine Längsverschiebung des zweiten Behälters 2 in Richtung hin zu der Nadel 52 bewirkt, um das Septum 22 des zweiten Behälters 2 mit der Nadel 52 anzustechen. Die Spiralfeder 42, die um die zweite Kolbenstange 41 herum angeordnet ist, befindet sich durch eine Klemmung zwischen der Innenseite des Kolbens 4 und dem Führungsbauteil 7 in Vorspannung, wobei ein freies Ende 411 der zweiten Kolbenstange 41 des zweiten Kolbens 4 durch das gemeinsame Haltebauteil 6 an Ort und Stelle gehalten wird, welches auch das freie Ende 311 der ersten Kolbenstange 31 des ersten Kolbens 3 hält. Entsprechend führt ein Loslösen der vorgespannten Spiralfeder 42 dazu, dass der zweite Kolben 4 bewegt wird und auf den Stopfen 21 trifft, zusammen mit diesem aufgrund des Staudrucks der in dem Behälter 2 angeordneten inkompressiblen Flüssigkeit den Behälter 2 axial gegen die Nadel 52 bewegt und dadurch die Fluidverbindung zwischen Leitungskanal 53 und Inhalt des Behälters 2 herstellt, welche dem Staudruck in Behälter 2 einen Ausgang bietet. Die Spiralfeder 42 drückt dann den Kolben 4 zusammen mit dem Stopfen 21 weiter und bewirkt damit, dass der Inhalt des zweiten Behälters 2 in den Leitungskanal 53, an dem Einwegventil 54 vorbei und weiter in den ersten Behälter 1 hinein befördert wird. Entsprechend wird bei der vorliegenden Ausführungsform die inkompressible Lösungsflüssigkeit, die als Inhalt in dem zweiten Behälter 2, d.h. in dem Lösungsflüssigkeitsbehälter 2 vorliegt, in den ersten Behälter 1, d.h. den Lyophilisatbehälter 1, gedrückt, in dem sich die Lösungsflüssigkeit mit dem Lyophilisat vermischt und damit eine Rekonstitution des Lyophilisats hervorruft. Die dadurch entstehende Lösung kann als abzugebende (zu injizierende) Substanz anschließend durch ein Auslösen der weiteren Spiralfeder 34 aus dem ersten Behälter 1 hinausbefördert und durch die (nicht gezeigte) Injektionseinrichtung nach außen abgegeben werden, vorzugsweise durch Autoinjektion an den Anwender selbst. An den Darstellungen in den Figuren 2 bis 5 kann bereits erkannt werden, dass die vorgespannte Spiralfeder 32 zum Anstechen des ersten Behälters 1 wesentlich kleiner dimensioniert ist als die beiden Spiralfedern 34 und 42, wodurch eine Auslenkung bzw. Längung der Spiralfeder 32 wesentlich kürzer als bei den anderen beiden Spiralfeder 34 und 42 ausfällt. Dies liegt daran, dass zum ausschließlichen Anstechen des ersten Behälters 1 durch die Spiralfeder 32 nur eine geringe axiale Verschiebung des Behälters 1 notwendig ist.

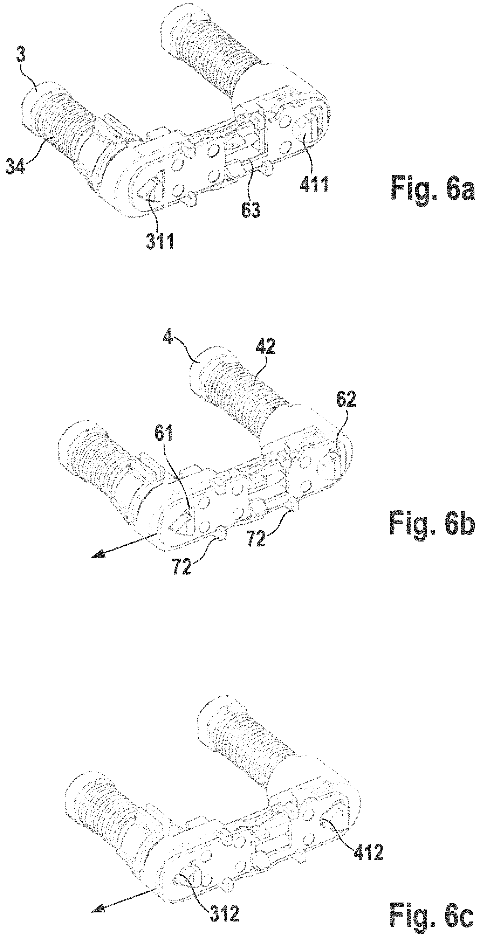

In den Figuren 6a bis 6c ist ein Vorgang des zeitlich versetzten Lösens zuerst der ersten vorgespannten Spiralfeder 32 und anschließend der zweiten vorgespannten Spiralfeder 42 dargestellt. In Figur 6a ist ein Ausgangszustand dargestellt, wie er ebenfalls in den Figuren 2 und 3 zu sehen ist. In diesem Ausgangszustand hält das gemeinsame Haltebauteil 6 die beiden freien Enden 311, 411 der beiden Kolbenstangen 31, 41 in Eingriff, so dass die beiden Spiralfeder 32, 42 in deren vorgespannten Zustand gehalten sind, und keine Bewegung weder der Kolben 3, 4 noch der Behälter 1, 2 stattfindet. Wie es unter anderem Fig. 6a entnommen werden kann weist das freie Ende 311 der ersten Kolbenstange 31 eine Dreiecksform auf. Eine zu dem freien Ende 311 der ersten Kolbenstange 31 entsprechende Aussparung 61, die in dem gemeinsamen Haltebauteil 6 für das Halten des freien Endes 311 der ersten Kolbenstange 31 vorgesehen ist, weist ebenfalls eine Dreiecksform auf. Dabei ist jedoch die Dreiecksform der Aussparung 61 größer als die Dreiecksform des freien Endes 311 der ersten Kolbenstange 31, wie es jeder der Figuren 6a bis 6c entnommen werden kann. In dem freien Ende 311 der ersten Kolbenstange 31 ist zudem eine Vertiefung in Gestalt einer Nut 312 vorgesehen, siehe auch Figur 3, in die ein Umfangsbereich der dreieckigen Aussparung 61 im in Figur 6a dargestellten Ausgangszustand eingeschoben ist. Ähnlich dazu weist das freie Ende 411 der zweiten Kolbenstange 41 eine halbrunde Form auf. Eine zu dem freien Ende 411 der zweiten Kolbenstange 41 entsprechende Aussparung 62, die in dem gemeinsamen Haltebauteil 6 für das Halten des freien Endes 411 der zweiten Kolbenstange 41 vorgesehen ist, weist ebenfalls eine halbrunde Form auf. Dabei ist jedoch wieder die halbrunde Form der Aussparung 62 größer als die halbrunde Form des freien Endes 411 der zweiten Kolbenstange 41, wie es jeder der Figuren 6a bis 6c entnommen werden kann. In dem freien Ende 411 der zweiten Kolbenstange 41 ist ebenfalls eine Vertiefung in Gestalt einer Nut 412 vorgesehen, siehe auch Figur 3, in die ein Umfangsbereich der halbrunden Aussparung 62 im in Figur 6a dargestellten Ausgangszustand eingeschoben ist. Der Größenunterschied zwischen den Aussparungen 61, 62 und den jeweiligen freien Enden 311, 411 ist dem Umstand geschuldet, dass die freien Enden 311, 411 nach einem jeweiligen Lösen durch die entsprechende Aussparung 61, 62 frei hindurchtreten sollen, ohne sich zu verkanten, also um den Lösevorgang der freien Enden 311, 411 sicherzustellen.

Bei der vorliegenden Ausführungsform ist das Verhältnis zwischen der Dreiecksform des freien Endes 311 der ersten Kolbenstange 31 und der Dreiecksform der Aussparung 61 so gewählt, dass deren Überdeckungsbereich im Ausgangszustand kleiner ist als ein Überdeckungsbereich zwischen der halbrunden Form des freien Endes 411 der zweiten Kolbenstange 41 und der halbrunden Form der Aussparung 62. Um nun ein Loslösen der beiden freien Enden 311, 411 der beiden Kolbenstangen 31, 41 hervorzurufen, kann das gemeinsame Haltebauteil 6 in dessen Längsrichtung, und damit im Wesentlichen senkrecht zu den Längsrichtungen der beiden Kolbenstangen 31, 41 bewegt werden, so dass das gemeinsame Haltebauteil 6 aus den Nuten 312, 412 herausgezogen wird. Dieser Herausziehvorgang tritt bei beiden freien Kolbenstangenenden 311, 411 durch das Herausziehen des gemeinsamen Haltebauteils 6 parallel zueinander auf. Aufgrund der Tatsache, dass bei der ersten Kolbenstange 31 der Überdeckungsbereich zwischen Nut 312 und Umgebung der Aussparung 61 kleiner ist als der Überdeckungsbereich zwischen Nut 412 und Umgebung der Aussparung 62 bei der zweiten Kolbenstange 41, führt das schrittweise Herausziehen des gemeinsamen Haltebauteils 6 zuerst zu einem Lösen des freien Endes 311 der ersten Kolbenstange 31, siehe auch Figur 6b, und damit zu einem Anstechen des ersten Behälters 1. Erst anschließend führt das weitere Herausziehen des gemeinsamen Haltebauteils 6 wie in Figur 6c gezeigt zu einem Lösen des freien Endes 411 der zweiten Kolbenstange 41, und damit zu einem Anstechen des zweiten Behälters 2 sowie einem anschließenden Drücken des Inhalts des zweiten Behälters 2 durch den Leitungskanal 53 vorbei an dem Einwegventil 54 hinein in den fluidverbundenen Behälter 1, was zur Rekonstitution des Lyophilisats führt. Dabei nimmt entsprechend der Inhalt des Behälters 1 um das hineingedrückte Volumen zu, so dass der Stopfen 11 des ersten Behälters 1 nach hinten in Richtung des ersten Kolbens 3 gedrückt wird. Dazu wird im Ausgangszustand, wie er in Figuren 2 und 3 gezeigt ist, zwischen dem Stopfen 11 und dem Kolben 3 ausreichend Platz gelassen, damit der Stopfen 11 Raum hat, sich nach hinten zu bewegen. Hierzu ist es offensichtlich notwendig, ein Entweichen der zurückgedrängten Luft vorbei an dem Kolben 3 zu ermöglichen, wie es unter anderem in Figur 3 gezeigt ist.

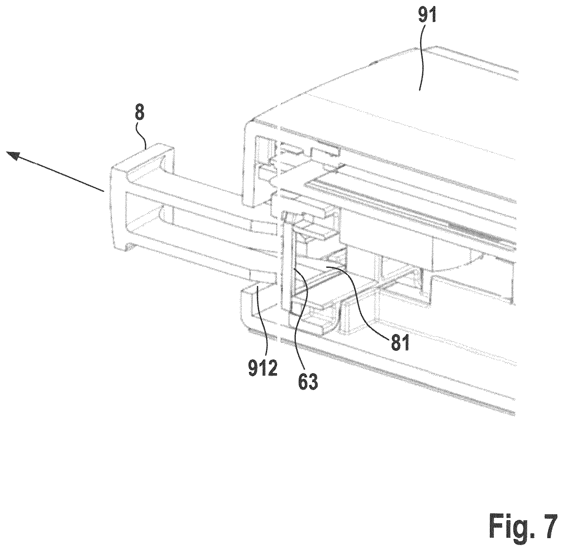

Bei genauer Betrachtung der Darstellungen in Figuren 6a bis 6c kann an einer Oberseite des gemeinsamen Haltebauteils 6 weiterhin eine Anordnung von drei Rastvertiefungen 64 erkannt werden, die durch eine an dem Führungsbauteil 7 einstückig angebrachte Rastnase 71 in vorzugsweise drei verschiedenen Positionen eingehakt werden können. Entsprechend dient die Kombination aus Rastvertiefungen 64 und Rastnase 71 dazu, die Bewegung des gemeinsamen Haltebauteils 6 bei der Anwendung der Vorrichtung 9 in einer prinzipiell horizontalen Richtung von einer Ausgangslage wie in Figur 6a dargestellt in eine Zwischenlage zum Auslösen der ersten Kolbenstange 31 wie in Figur 6b dargestellt bis in eine Endlage zum Auslösen der zweiten Kolbenstange 41 stufenweise einrastend zu führen, also in den angesprochenen drei Positionen. Die generelle Bewegung des gemeinsamen Haltebauteils 6 wird, wie hier dargestellt, durch Führungsschienen 72 geführt, welche an dem Führungsbauteil 7 angebracht sind. Wie es schließlich in Figur 7 dargestellt ist, weist die Vorrichtung 9, insbesondere bei der vorliegenden bevorzugten Ausführungsform eine Innenseite der Abdeckung 92, ein eventuell damit verbundenes Aktivierungsbauteil 8 auf, das im zusammengebauten Zustand der Vorrichtung 9 durch die Öffnung 912 in dem Hauptkörper 91 sowie durch eine Aussparung 63 bzw. eine Aktivierungsaussparung 63 in dem gemeinsamen Haltebauteil 6 hindurchtritt. Das Führungsbauteil 7 hat zudem eine zu der Aktivierungsaussparung 63 entsprechende Aussparung (nicht gezeigt), um einen Durchtritt des Aktivierungsbauteils 8 durch die Öffnung 912, die Aktivierungsaussparung 63 sowie die (nicht gezeigte) Führungsbauteilaussparung zu ermöglichen. Das Aktivierungsbauteil 8, das vorzugsweise durch zwei Längsstege ausgebildet ist, weist dabei einen Vorsprung 81 in Form einer Kaskadenfläche an jeder Stegvorderseite so auf, dass ein Herausziehen des Aktivierungsbauteils 8 in dessen axialer Richtung ein Drücken des gemeinsamen Haltebauteils 6 in die axiale Richtung des gemeinsamen Haltebauteils 6, die im Wesentlichen senkrecht zu der axialen Richtung des Aktivierungsbauteils 8 steht, auslöst, wodurch das gemeinsame Haltebauteil 6 zum Lösen der beiden freien Enden 311, 411 der beiden Kolbenstangen 31, 41 bewegt wird. Entsprechend ist bei der vorliegenden bevorzugten Ausführungsform eine Längsachse des Aktivierungsbauteils 8 im Wesentlichen senkrecht zu der Längsachse des gemeinsamen Haltebauteils 6 angeordnet, d.h. im Wesentlichen parallel zu den Längsachsen der beiden Kolbenstangen 31, 41 und damit der beiden Behälter 1, 2, wobei eine Herausziehrichtung des Aktivierungsbauteils 8 in dessen Längsrichtung vorzugsweise im Wesentlichen senkrecht bzw. im Wesentlichen im rechten Winkel zu der Bewegungsrichtung des gemeinsamen Haltebauteils 6 in dessen Längsrichtung verläuft. Durch die Kombination aus Kaskadenfläche und Aktivierungsaussparung stehen entsprechend das Aktivierungsbauteil 8 und das gemeinsame Haltebauteil 6 in mechanischer Wirkverbindung, so dass die Bewegung des einen Bauteils, beispielsweise des Aktivierungsbauteils 8, eine Bewegung des anderen Bauteils, in diesem Fall des gemeinsamen Haltebauteils 6, bewirkt.

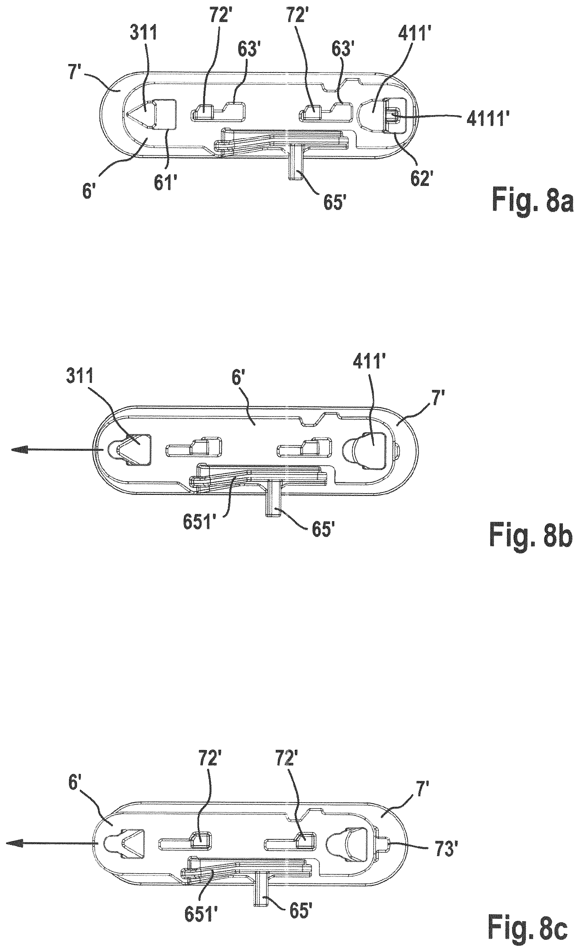

In den Figuren 8a bis 8c ist eine alternative Ausführung einer Kombination aus Führungsbauteil 7' und gemeinsamen Haltebauteil 6' dargestellt, wobei die Figuren 8a bis 8c ähnlich wie in den Figuren 6a bis 6c gezeigt einen Vorgang des zeitlich versetzten Lösens zuerst der ersten vorgespannten Spiralfeder 32 und anschließend der zweiten vorgespannten Spiralfeder 42 darstellen. Der grundsätzliche Ablauf des Lösevorgang bleibt im Vergleich zu der vorhergehend beschriebenen Ausführungsform im Wesentlichen unverändert, wobei lediglich die Betätigung des gemeinsamen Haltebauteils 6' sowie die Führung des gemeinsamen Haltebauteils 6' an dem Führungsbauteil 7'auf eine andere Art und Weise erfolgt, wie es nachfolgend näher beschrieben ist.

In Bezug auf die alternative Führung des gemeinsamen Haltebauteils 6' an dem Führungsbauteil 7' wird bei der vorliegend beschriebenen Ausführungsform das gemeinsame Haltebauteil 6' anstelle von den dazu außen angeordneten Führungsschienen 72 hier durch zwei innen angeordnete Führungsschienen 72' geführt, die jeweils in einer weiter innen angeordneten Aussparung 63'verlaufen beispielsweise in Form einer durchgehenden Führungsnut. Die Führungsschienen 72' weisen jeweils im Wesentlichen die Form eines auf dem Kopf stehenden Buchstaben L auf (= Γ) und erstrecken sich jeweils von einer nach außen gerichteten Oberfläche des Führungsbauteils 7' weg in Richtung des gemeinsamen Haltebauteils 6' hin und durch dieses hindurch, genauer gesagt durch die jeweilige Aussparung 63' hindurch. Jede Aussparung 63' zeigt dazu an einem ersten, in den Figuren auf der rechten Seite dargestellten Ende eine sogenannte Durchführungserweiterung zum freien Hindurchführen der jeweiligen Führungsschiene 72', sowie eine an die Durchführungserweiterung anschließende und entsprechend verengte Führungsnut, welche den axial verlaufenden, längeren Abschnitt der jeweiligen L-förmigen Führungsschiene 72' aufnimmt und diesen führt.

Bezüglich der alternativen Betätigung des gemeinsamen Haltebauteils 6' ist in Figur 8a ein Ausgangszustand vor dem Lösevorgang dargestellt, bei dem das gemeinsame Haltebauteil 6' das freie Ende 311 der ersten Kolbenstage 31 sowie eine alternative Ausführung eines freien Endes 411' der zweiten Kolbenstage 41 in Eingriff hält, so dass die beiden Spiralfeder 32, 42 in deren vorgespannten Zuständen gehalten werden und entsprechend keine Bewegung weder der Kolben 3, 4 noch der Behälter 1, 2 stattfinden kann. Die vorgenannte alternative Ausführung des freien Endes 411' der zweiten Kolbenstage 41 unterscheidet sich von der vorhergehend beschriebenen Ausführung des freien Endes 411 der zweiten Kolbenstange 41 insbesondere dadurch, dass dieses zusätzlich einen nach außen vorstehenden Vorsprung 4111' in Form eines Stegs oder dergleichen aufweist, welcher zu dem runden Anteil der im Wesentlichen halbrunden Form des freien Endes 411' der zweiten Kolbenstage 41 entgegengesetzt angeordnet ist. Der Vorsprung 4111', auch als Verdrehsicherung beziehungsweise Verdrehsicherungsvorsprung bezeichnet, verläuft in einer entsprechenden Nut 73', welche in dem Führungsbauteil 7' vorgesehen ist und an die Form des Vorsprungs 4111' angepasst ist, und dient dazu, ein Verdrehen des freien Endes 411' der zweiten Kolbenstange 41, und damit der gesamten zweiten Kolbenstage 41 zu verhindern, wobei ein derartiges Verdrehen beispielsweise bei einem Zusammenbau der erfindungsgemäßen Vorrichtung oder auch bei einer erschütterungsbedingten Vibration beim Transport der erfindungsgemäßen Vorrichtung auftreten kann.

Wie es unter anderem Figur 8a entnommen werden kann weist das freie Ende 311 der ersten Kolbenstange 31 ähnlich wie in der oben beschriebenen Ausführungsform ebenfalls eine Dreiecksform auf, welche bereits für sich genommen formbedingt eine Verdrehsicherung bietet. Eine zu dem freien Ende 311 der ersten Kolbenstange 31 entsprechende Aussparung 61', die in dem gemeinsamen Haltebauteil 6' für das Halten des freien Endes 311 der ersten Kolbenstange 31 vorgesehen ist, weist eine im Wesentlichen entsprechende, dreieckig anmutende Form auf, bei der eine vordere Spitze abgerundet ausgebildet ist, um mit der beispielsweise halbrunden Form der Nut 312 übereinzustimmen. Bei der vorliegenden alternativen Ausführungsform ist das Verhältnis zwischen der Dreiecksform des freien Endes 311 der ersten Kolbenstange 31 und der im Wesentlichen dreieckigen Form der Aussparung 61' ähnlich wie bei der vorhergehend beschriebenen Ausführungsform so gewählt, dass deren Überdeckungsbereich im Ausgangszustand kleiner als ein Überdeckungsbereich zwischen der halbrunden Form des freien Endes 411' der zweiten Kolbenstange 41 und der halbrunden Form der Aussparung 62' ist . Um nun ein Loslösen der beiden freien Enden 311, 411' der beiden Kolbenstangen 31, 41 zu bewirken kann das gemeinsame Haltebauteil 6' in dessen Längsrichtung, und damit im Wesentlichen senkrecht zu den Längsrichtungen der beiden Kolbenstangen 31, 41 bewegt werden, so dass das gemeinsame Haltebauteil 6' aus den Nuten 312, 412 herausgezogen wird. Dieses Herausziehen des gemeinsamen Haltebauteils 6' aus den Nuten 312, 412 wird auch als Lösevorgang bezeichnet. Der Lösevorgang erfolgt bei beiden freien Kolbenstangenenden 311, 411' durch das in den Figuren 8a bis 8c dargestellte Ziehen des gemeinsamen Haltebauteils 6' in der Figurenebene nach links, siehe auch die in Figuren 8b und 8c dargestellten Pfeile. Aufgrund der Tatsache, dass bei der ersten Kolbenstange 31 der Überdeckungsbereich zwischen Nut 312 und Umgebung der Aussparung 61'ähnlich wie bei der vorhergehend beschriebenen Ausführungsform kleiner als der Überdeckungsbereich zwischen Nut 412 und Umgebung der Aussparung 62' bei dem alternativen freien Ende 411' der zweiten Kolbenstange 41 ist, führt das dargestellte schrittweise Herausziehen des gemeinsamen Haltebauteils 6' zuerst zu einem Lösen / Freigeben des freien Endes 311 der ersten Kolbenstange 31, wie in Figur 8b dargestellt, und damit zu einem Anstechen des ersten Behälters 1. Erst anschließend führt das weitere Bewegen des gemeinsamen Haltebauteils 6' wie in Figur 8c dargestellt zu einem Lösen / Freigeben des freien Endes 411' der zweiten Kolbenstange 41, und damit zu einem Anstechen des zweiten Behälters 2 sowie einem anschließenden Drücken des Inhalts des zweiten Behälters 2 durch den Leitungskanal 53 vorbei an dem Einwegventil 54 hinein in den fluidverbundenen Behälter 1, was zur Rekonstitution des Lyophilisats führt.

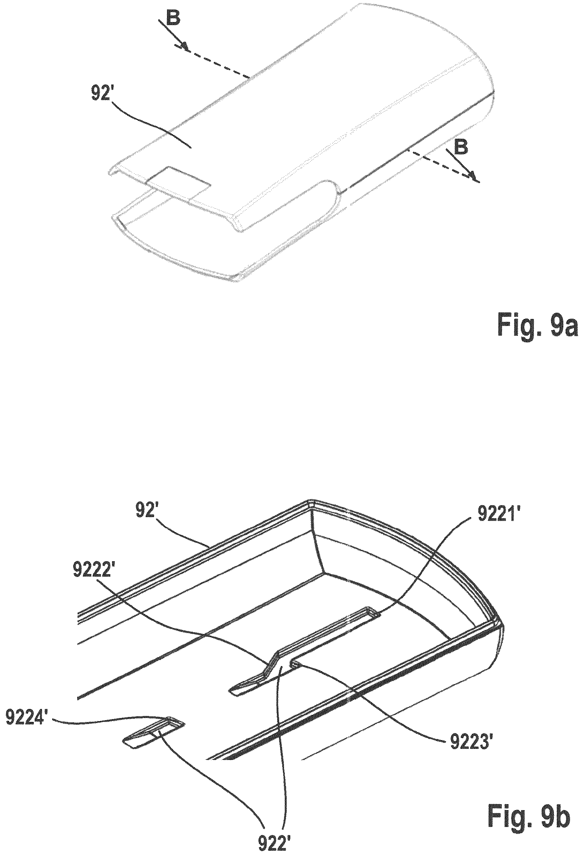

Bei genauer Betrachtung der Darstellungen in Figuren 8a bis 8c kann an einer Unterseite des gemeinsamen Haltebauteils 6' ein Betätigungselement 65' in Gestalt eines nach außen vorstehenden Stifts oder Bolzens erkannt werden, der über einen Federmechanismus 651' mit dem gemeinsamen Haltebauteil 6' verbunden ist. Durch eine Betätigung/Bewegung des stiftförmigen Betätigungselements 65' wird die gewünschte Bewegung des gemeinsamen Haltebauteils 6' und damit der vorhergehend beschriebene Lösevorgang der beiden freien Enden 311, 411' der Kolbenstangen 31, 41 bewirkt. Um nun das Betätigungselement 65' entsprechend zu betätigen ist an einer Innenseite einer alternativen Ausführungsform einer Abdeckung 92', wie sie in Figuren 9a und 9b gezeigt ist, eine sogenannte Führungs- oder Steuerungsnut 922' vorgesehen, die bei der vorliegend beschriebenen Ausführungsform beispielsweise zweiteilig ausgebildet ist. In einem zusammengebauten Zustand der Vorrichtung 9 ist entsprechend das Betätigungselement 65' innerhalb eines ersten Abschnitts der Steuerungsnut 922' angeordnet. In einem Ausgangszustand, wie er in Fig. 8a gezeigt ist, befindet sich das Betätigungselement 65' entsprechend an einem ersten Ende 9221' des längeren, ersten Abschnitts der Steuerungsnut 922', wie es in Figur 9b auf einer rechten Seite der Innenseite der unteren Hälfte der alternativen Abdeckung 92' dargestellt ist. Bei einem Entfernen der Abdeckung 92' von dem Hauptkörper 91 der Vorrichtung 9 verläuft eine entsprechende Bewegung des Betätigungselements 65' weg von dem ersten Ende 9221' hin zu einer nachfolgend angeordneten Schrägfläche bzw. Schräge 9222' der Steuerungsnut 922'. Durch ein sich Entlangbewegen des Betätigungselements 65' an dieser Schräge 9222', bewirkt durch das weitere Abziehen der Abdeckung 92' von dem Hauptkörper 91, erfolgt die Bewegung des mit dem Betätigungselements 65' einstückig verbundenen Haltebauteils 6', wie sie in den Figuren 8a bis 8c dargestellt ist. Nach vollständigem Bewegen des Betätigungselements 65' entlang der gesamten Schräge 9222' wird letztendlich der in Figur 8c dargestellte Zustand erreicht, in dem beide freien Enden 311, 411' der Kolbenstangen 31, 41 von dem Haltebauteil 6' losgelöst sind.

Ab einem gewissen Zustand der Loslösebewegung, genauer gesagt ab dem Lösen des freien Endes 311 der ersten Kolbenstange 31 des ersten Kolbens 3, darf das gemeinsame Haltebauteil 6' nicht mehr in seine ursprüngliche Ausgangslage zurückbewegbar sein, um zu verhindern, dass ein Anwender den Eindruck einer unbenutzten Vorrichtung 9 bekommt, obwohl bereits wesentliche Rekonstitutionsvorgänge stattgefunden haben. Um in diesem Sinne nun ein Zurückbewegen des Betätigungselements 65' und damit des gemeinsamen Haltebauteils 6' durch ein versehentliches oder absichtliches Zurückschieben der Abdeckung 92' zu verhindern, weist die Steuerungsnut 922' an einer der Schräge 9222' gegenüberliegenden Seite einen ersten Anschlag 9223' auf, an dem das Betätigungselement 65' in dem Fall des Zurückschiebens der Abdeckung 92' in Anlage geht und damit eine weiterführende Zurückbewegen des Betätigungselements 65' verhindert.

Bei einem gewünschten Fortschreiten des Abziehvorgangs der Abdeckung 92' von dem Hauptkörper 91 verlässt das Betätigungselement 65' nach der Schräge 9222 ' und beispielsweise unterstützt durch eine Rampe oder dergleichen den ersten Abschnitt der Steuerungsnut 922', und tritt nachfolgend optional in einen kürzeren, zweiten Abschnitt der Steuerungsnut 922' ein, wie er auf der linken Seite in Fig. 9b dargestellt ist. Dieser kürzere Abschnitt der Steuerungsnut 922' weist ähnlich wie der erste, längere Abschnitt der Steuerungsnut 922' inklusive der Schräge 9222' einen zweiten Anschlag 9224' auf, an dem das Betätigungselement 65' bei Zurückschieben der Abdeckung 92' nach einem Fortschreiten des Abziehvorgangs der Abdeckung 92' von dem Hauptkörper 91 in Anlage gehen kann und damit eine Zurückbewegen des Betätigungselements 65' ab diesem Punkt sicher verhindert. Bei weiterer Ausführung des Abziehvorgangs der Abdeckung 92' von dem Hauptkörper 91 verlässt das Betätigungselement 65'beispielsweise unterstützt durch eine Rampe oder dergleichen den zweiten Abschnitt der Steuerungsnut 922', und der Hauptkörper 91 verlässt anschließend vollständig die Abdeckung 92', um die durch eine Versiegelung abgedeckte Öffnung der Injektionseinrichtung freizugeben, so dass die durch die Injektionseinrichtung abzugebende rekonstituierte Substanz ausgestoßen werden kann.

Vorhergehend wurden bevorzugte Ausführungsformen der vorliegenden Erfindung beschrieben, wobei aber die vorliegende Erfindung nicht auf die zuvor beschriebenen bevorzugten Ausführungsformen beschränkt ist. Diverse Modifikationen in der Ausgestaltung können vorgenommen werden, ohne von der Erfindung abzuweichen, wie sie im Umfang der nachfolgenden Ansprüche vorgegeben ist.

### Bezugszeichenliste

- A: erste Substanz (z.B. ein Lyophilisat)
- B: zweite Substanz (z. B. eine Rekonstitutionsflüssigkeit)
- 1: erster Behälter
- 11: Stopfen des ersten Behälters
- 12: Septum des ersten Behälters
- 2: zweiter Behälter
- 21: Stopfen des zweiten Behälters
- 22: Septum des zweiten Behälters
- 3: erster Kolben
- 31: erste Kolbenstange
- 311: freies Ende der ersten Kolbenstange
- 312: Nut im freien Ende der ersten Kolbenstange
- 32: erstes vorgespanntes Federelement für erste Kolbenstange
- 33: Zwischenbauteil
- 34: weiteres Federelement
- 4: zweiter Kolben
- 41: zweite Kolbenstange
- 411: freies Ende der zweiten Kolbenstange
- 411': freies Ende der zweiten Kolbenstange (alternative Ausführung)
- 4111': Verdrehsicherungsvorsprung des freien Endes der zweiten Kolbenstange
- 412: Nut im freien Ende der zweiten Kolbenstange
- 42: zweites vorgespanntes Federelement für zweite Kolbenstange
- 5: Leitungseinrichtung
- 51: erste Nadel
- 52: zweite Nadel
- 53: Leitungskanal
- 54: Einwegventil (z.B. ein Gummilippen-Rückschlagventil)
- 6: gemeinsames Haltebauteil / Trigger
- 6': gemeinsames Haltebauteil / Trigger (alternative Ausführung)
- 61: erste Aussparung für freies Ende der ersten Kolbenstange
- 61': erste Aussparung für freies Ende der ersten Kolbenstange (alternative Ausführung)
- 62: zweite Aussparung für freies Ende der zweiten Kolbenstange
- 62': zweite Aussparung für freies Ende der zweiten Kolbenstange (alternative Ausführung)
- 63: Aussparung für Aktivierungsbauteil
- 63': Aussparungen für Führungsschienen
- 64: Rastvertiefungen
- 65': Triggerbetätigungselement
- 651': Federmechanismus
- 7: Führungsbauteil
- 7': Führungsbauteil (alternative Ausführung)
- 71: Rastnase
- 72: Führungsschienen
- 72': Führungsschienen (alternative Ausführung)
- 73': Verdrehsicherungsnut
- 8: Aktivierungsbauteil
- 81: Vorsprung in Form einer Kaskadenfläche
- 9: Vorrichtung
- 91: Hauptkörper
- 911: Griffleiste
- 912: Öffnung
- 92: Abdeckung
- 92': Abdeckung (alternative Ausführung)
- 921: Aussparung in der Abdeckung
- 922': Steuerungsnut
- 9221': erstes Ende der Steuerungsnut
- 9222': Schräge der Steuerungsnut
- 9223': erster Anschlag der Steuerungsnut
- 9224': zweiter Anschlag der Steuerungsnut

## Patentansprüche

1. Vorrichtung (9) zur Abgabe mindestens einer Substanz, insbesondere an einen Patienten, umfassend
einen eine erste Substanz (A) enthaltenden ersten Behälter (1) mit einem ersten Kolben (3) mit einer ersten Kolbenstange (31),
einen eine zweite Substanz (B) enthaltenden zweiten Behälter (2) mit einem zweiten Kolben (4) mit einer zweiten Kolbenstange (41),
eine Injektionseinrichtung, und
eine Leitungseinrichtung (5) zum Leiten des Inhalts des zweiten Behälters (2) zum ersten Behälter (1) zum dortigen Mischen und des Inhalts des ersten Behälters (1) zur Injektionseinrichtung,
wobei der erste und der zweite Behälter (2) zur Herstellung einer Fluidverbindung über ein jeweiliges erstes und zweites Anschlusselement (51, 52) mit der Leitungseinrichtung (5) in der Vorrichtung (9) beweglich angeordnet sind,
wobei der erste und der zweite Kolben (3, 4) jeweils über mindestens ein vorgespanntes Federelement (32, 34, 42) bewegbar sind,
wobei ein freies Ende (311) der ersten Kolbenstange (31) und ein freies Ende (411) der zweiten Kolbenstange (41) durch ein gemeinsames Haltebauteil (6) lösbar gehalten sind, und
wobei das gemeinsame Haltebauteil (6) derart gestaltet ist, dass eine Bewegung des gemeinsamen Haltebauteils (6) zu einem nacheinanderfolgenden Lösen der ersten Kolbenstange (31) und der zweiten Kolbenstange (41) und einer Bewegung der ersten Kolbenstange (31) und der zweiten Kolbenstange (41) im Wesentlichen senkrecht zu der Bewegungsrichtung des gemeinsamen Haltebauteils (6) führt, wobei nacheinanderfolgend, durch die Bewegung der ersten Kolbenstange (31) der erste Behälter (1) über das erste Anschlusselement (51) und über die Leitungseinrichtung (5) in Fluidverbindung gebracht wird mit dem zweiten Behälter (2) über das zweite Anschlusselement (52) durch die darauffolgende Bewegung der zweiten Kolbenstange (41), wobei die erste Substanz (A) mit der zweiten Substanz (B) gemischt wird, wobei die Leitungseinrichtung (5) ein Einwegventil (54) beinhaltet, das Fluidfluss von dem zweiten Behälter (2) in den ersten Behälter (1) zulässt, jedoch einen Rückfluss eines Fluids von dem ersten Behälter (1) in den zweiten Behälter (2) verhindert.

2. Vorrichtung (9) nach Anspruch 1, wobei das gemeinsame Haltebauteil (6), welches vorzugsweise im Wesentlichen plattenförmig ausgebildet ist, die erste Kolbenstange (31) und die zweite Kolbenstange (41) gegen die Vorspannung des jeweiligen vorgespannten Federelements (32, 42) zurückhält.

3. Vorrichtung (9) nach einem der vorangehenden Ansprüche, wobei
das gemeinsame Haltebauteil (6) zumindest eine Aussparung (61) für das freie Ende (311) der ersten Kolbenstange (31) und zumindest eine Aussparung (62) für das freie Ende (411) der zweiten Kolbenstange (41) aufweist,
das freie Ende (311) der ersten Kolbenstange (31) und das freie Ende (411) der zweiten Kolbenstange (41) jeweils eine Vertiefung (312, 412) aufweisen, welche mit dem gemeinsamen Haltebauteil (6) lösbar in Eingriff ist, und
eine Größe des freien Endes (311, 411) der ersten und der zweiten Kolbenstange (31, 41) sowie eine Größe der jeweiligen Aussparung (61, 62) und/oder eine Tiefe der jeweiligen Vertiefung (312, 412) derart gewählt ist, dass die freien Enden (311, 411) der ersten und der zweiten Kolbenstange (31, 41) unterschiedlich mit dem gemeinsamen Haltebauteil (6) zusammenwirken.

4. Vorrichtung (9) nach Anspruch 3, wobei
die Aussparung (61) in dem gemeinsamen Haltebauteil (6) für das freie Ende (311) der ersten Kolbenstange (31) im Wesentlichen eine Dreiecksform und die Aussparung (62) in dem gemeinsamen Haltebauteil (6) für das freie Ende (411) der zweiten Kolbenstange (41) im Wesentlichen eine halbrunde Form aufweist, und das freie Ende (311) der ersten Kolbenstange (31) im Wesentlichen eine Dreiecksform und das freie Ende (411) der zweiten Kolbenstange (41) im Wesentlichen eine halbrunde Form aufweist, und/oder
die Tiefe der Vertiefung (412) in dem freien Ende (411) der zweiten Kolbenstange (41) tiefer als die Tiefe der Vertiefung (312) in dem freien Ende (311) der ersten Kolbenstange (31) ist.

5. Vorrichtung (9) nach einem der vorangehenden Ansprüche, wobei das mindestens eine vorgespannte Federelement (32) des ersten Kolbens (3) nach einem Lösen der ersten Kolbenstange (31) den ersten Kolben (3) zusammen mit dem ersten Behälter (1) gegenüber der Vorrichtung (9) bewegt, und das mindestens eine vorgespannte Federelement (42) des zweiten Kolbens (4) nach einem Lösen der zweiten Kolbenstange (41) den zweiten Kolben (4) zusammen mit dem zweiten Behälter (2) gegenüber der Vorrichtung (9) und darauffolgend den zweiten Kolben (4) gegenüber dem zweiten Behälter (2) bewegt.

6. Vorrichtung (9) nach einem der vorangehenden Ansprüche, wobei die erste Substanz (A) von der zweiten Substanz (B) verschieden ist.

7. Vorrichtung (9) nach einem der vorangehenden Ansprüche, wobei die zweite Substanz (B) eine Flüssigkeit ist, vorzugsweise wobei durch Verschieben des zweiten Kolbens (4) gegen ein Behälterende des zweiten Behälters (2) die Flüssigkeit in den ersten Behälter (1) übertragen wird, die Flüssigkeit sich dort mit der ersten Substanz (A) mischt und diese eine Lösung ausbilden, und wobei die Lösung in dem ersten Behälter (1) durch Verschieben des ersten Kolbens (3) gegen ein Behälterende des ersten Behälters (1) über die Injektionseinrichtung abgebbar ist.

8. Vorrichtung (9) nach Anspruch 7, wobei die erste Substanz (A) ein Feststoff ist, insbesondere ein Lyophilisat.

9. Vorrichtung (9) nach einem der vorangehenden Ansprüche, wobei das Lösen des freien Endes (311) der ersten Kolbenstange (31) aufgrund des entsprechenden Federelements (32) eine Fluidverbindung zwischen der Leitungseinrichtung (5) und dem ersten Behälter (1) herstellt, und wobei ein darauffolgendes Lösen des freien Endes (411) der zweiten Kolbenstange (41) aufgrund des entsprechenden Federelements (42) eine Fluidverbindung zwischen der Leitungseinrichtung (5) und dem zweiten Behälter (2) herstellt und zusätzlich eine Übertragung der zweiten Substanz (B) aus dem zweiten Behälter (2) über die Leitungseinrichtung (5) in den ersten Behälter (1) bewirkt.

10. Vorrichtung (9) nach einem der vorangehenden Ansprüche, wobei eine Längsachse des gemeinsamen Haltebauteils (6) im Wesentlichen senkrecht zu den Längsachsen der ersten und der zweiten Kolbenstange (31, 41) angeordnet ist.

11. Vorrichtung (9) nach einem der vorangehenden Ansprüche, wobei die Vorrichtung (9) ferner ein Aktivierungsbauteil (8) aufweist, das mit dem gemeinsamen Haltebauteil (6) in mechanischer Wirkverbindung steht, und wobei eine Bewegung des Aktivierungsbauteils (8) zu der Bewegung des gemeinsamen Haltebauteils (6) führt, vorzugsweise wobei eine Längsachse des Aktivierungsbauteils (8) im Wesentlichen senkrecht zu der Längsachse des gemeinsamen Haltebauteils (6) angeordnet ist, und wobei eine Bewegungsrichtung des Aktivierungsbauteils (8) vorzugsweise im Wesentlichen senkrecht zu der Bewegungsrichtung des gemeinsamen Haltebauteils (6) verläuft.

12. Vorrichtung (9) nach einem der vorangehenden Ansprüche, wobei die Leitungseinrichtung (5) ein erstes Anschlusselement (51) für eine Fluidverbindung mit dem ersten Behälter (1) und ein zweites Anschlusselement (52) für eine Fluidverbindung mit dem zweiten Behälter (2) aufweist, und wobei jedes Anschlusselement (51, 52) vorzugsweise als ein nadelartiger Vorsprung zum Durchdringen eines Septums (12, 22) des jeweiligen Behälters (1, 2) ausgeführt ist.

13. Vorrichtung (9) nach einem der vorangehenden Ansprüche, wobei die Leitungseinrichtung (5) eine Fluidverbindung zu der Injektionseinrichtung aufweist, und/oder wobei die Leitungseinrichtung (5) vorzugsweise ein Ventilelement (54) aufweist, weiter vorzugsweise ein Einwegventil (54), insbesondere ein Rückschlagventil (54).

14. Vorrichtung (9) nach einem der vorangehenden Ansprüche, wobei der erste Behälter (1) ein erstes Behälterende und ein zweites Behälterende aufweist, und der zweite Behälter (2) ein erstes Behälterende und ein zweites Behälterende aufweist, wobei der erste und der zweite Kolben (3, 4) zwischen dem jeweiligen ersten Behälterende und dem jeweiligen zweiten Behälterende verschiebbar sind, und wobei durch Verschieben des ersten Kolbens (3) gegen das erste Behälterende des ersten Behälters (1) ein Inhalt des ersten Behälters (1) über die Injektionseinrichtung abgebbar ist.

15. Vorrichtung (9) nach einem der vorangehenden Ansprüche, wobei
jeder Behälter (1, 2) zylinderförmig ausgebildet ist, und/oder
der zweite Behälter (2) parallel, insbesondere gleichgerichtet, zum ersten Behälter (1) in der Vorrichtung (9) angeordnet ist.

16. Verfahren zum Bereitstellen einer gemischten Substanz mit einer Vorrichtung (9) nach einem der vorangehenden Ansprüche, umfassend einen Schritt des Bewegens des mit dem gemeinsamen Haltebauteil (6) in Wirkverbindung stehenden Aktivierungsbauteils (8) entlang dessen Längsachse, wodurch das gemeinsamen Haltebauteil (6) im Wesentlichen in dessen Längsrichtung bewegt wird, insbesondere senkrecht zu der Bewegungsrichtung des Aktivierungsbauteils (8), und dabei zuerst den Eingriff zwischen dem gemeinsamen Haltebauteil (6) und dem freien Ende (311) der ersten Kolbenstange (31) und darauffolgend den Eingriff zwischen dem gemeinsamen Haltebauteil (6) und dem freien Ende (411) der zweiten Kolbenstange (41) löst, vorzugsweise wobei das Lösen des freien Endes (311) der ersten Kolbenstange (31) aufgrund des entsprechenden Federelements (32) eine Fluidverbindung zwischen der Leitungseinrichtung (5) und dem ersten Behälter (1) bewirkt, und das darauffolgende Lösen des freien Endes (411) der zweiten Kolbenstange (41) aufgrund des entsprechenden Federelements (42) eine Fluidverbindung zwischen der Leitungseinrichtung (5) und dem zweiten Behälter (2) bewirkt und anschließend die zweite Substanz (B) aus dem zweiten Behälter (2) über die Leitungseinrichtung (5) in den ersten Behälter (1) übertragen wird.

17. Verfahren nach Anspruch 16, wobei die erste Substanz (A) ein Feststoff, insbesondere ein Lyophilisat, und die zweite Substanz (B) eine Flüssigkeit ist, und wobei durch Verschieben des zweiten Kolbens (4) gegen das erste Behälterende des zweiten Behälters (2) die Flüssigkeit in den ersten Behälter (1) übertragen wird, die Flüssigkeit sich dort mit dem Feststoff verbindet und diese eine Lösung ausbilden.

## Claims

1. Device (9) for dispensing at least one substance, in particular to a patient, comprising
a first container (1) which contains a first substance (A) and has a first piston (3) having a first piston rod (31),
a second container (2) which contains a second substance (B) and has a second piston (4) having a second piston rod (41),
an injection apparatus, and
a conduit apparatus (5) for conveying the contents of the second container (2) to the first container (1) for mixing therein, and for conveying the contents of the first container (1) to the injection apparatus,
wherein the first and the second container (2) are movably arranged in the device (9) to establish a fluid connection, via a relevant first and second connection element (51, 52), with the conduit apparatus (5),
wherein the first and the second piston (3, 4) can each be moved via at least one preloaded spring element (32, 34, 42),
wherein a free end (311) of the first piston rod (31) and a free end (411) of the second piston rod (41) are releasably held by a common holding component (6), and
wherein the common holding component (6) is designed such that a movement of the common holding component (6) leads to a successive release of the first piston rod (31) and the second piston rod (41) and to a movement of the first piston rod (31) and the second piston rod (41) substantially perpendicular to the direction of movement of the common holding component (6), wherein successively, by means of the movement of the first piston rod (31), the first container (1), via the first connection element (51) and via the conduit apparatus (5), is brought into fluid communication with the second container (2), via the second connection element (52), by means of the subsequent movement of the second piston rod (41), wherein the first substance (A) is mixed with the second substance (B), wherein the conduit apparatus (5) includes a one-way valve (54) which allows fluid flow from the second container (2) into the first container (1), but prevents backflow of a fluid from the first container (1) into the second container (2).

2. Device (9) according to claim 1, wherein the common holding component (6), which is preferably substantially planar, holds back the first piston rod (31) and the second piston rod (41) against the preloading of the relevant preloaded spring element (32, 42).

3. Device (9) according to either of the preceding claims, wherein
the common holding component (6) has at least one recess (61) for the free end (311) of the first piston rod (31) and at least one recess (62) for the free end (411) of the second piston rod (41),
the free end (311) of the first piston rod (31) and the free end (411) of the second piston rod (41) each have a depression (312, 412) which is releasably engaged with the common holding component (6), and
a size of the free end (311, 411) of the first and the second piston rod (31, 41) as well as a size of the relevant recess (61, 62) and/or a depth of the relevant depression (312, 412) is selected such that the free ends (311, 411) of the first and the second piston rod (31, 41) interact differently with the common holding component (6).

4. Device (9) according to claim 3, wherein
the recess (61) in the common holding component (6) for the free end (311) of the first piston rod (31) has a substantially triangular shape and the recess (62) in the common holding component (6) for the free end (411) of the second piston rod (41) has a substantially semicircular shape, and the free end (311) of the first piston rod (31) has a substantially triangular shape and the free end (411) of the second piston rod (41) has a substantially semicircular shape, and/or
the depth of the depression (412) in the free end (411) of the second piston rod (41) is deeper than the depth of the depression (312) in the free end (311) of the first piston rod (31).

5. Device (9) according to any of the preceding claims, wherein the at least one preloaded spring element (32) of the first piston (3) moves the first piston (3) together with the first container (1) relative to the device (9) after the first piston rod (31) has been released, and the at least one preloaded spring element (42) of the second piston (4) moves the second piston (4) together with the second container (2) relative to the device (9), and subsequently the second piston (4) relative to the second container (2), after the second piston rod (41) has been released.

6. Device (9) according to any of the preceding claims, wherein the first substance (A) is different from the second substance (B).

7. Device (9) according to any of the preceding claims, wherein the second substance (B) is a liquid, preferably wherein the liquid is transferred into the first container (1) by pushing the second piston (4) against a container end of the second container (2), the liquid is mixed with the first substance (A) in the first container and they form a solution, and wherein the solution in the first container (1) can be dispensed via the injection apparatus by pushing the first piston (3) against a container end of the first container (1).

8. Device (9) according to claim 7, wherein the first substance (A) is a solid, in particular a lyophilizate.

9. Device (9) according to any of the preceding claims, wherein the release of the free end (311) of the first piston rod (31) establishes a fluid connection between the conduit apparatus (5) and the first container (1) due to the corresponding spring element (32), and wherein a subsequent release of the free end (411) of the second piston rod (41) establishes a fluid connection between the conduit apparatus (5) and the second container (2) due to the corresponding spring element (42) and additionally causes a transfer of the second substance (B) from the second container (2), via the conduit apparatus (5), into the first container (1).

10. Device (9) according to any of the preceding claims, wherein a longitudinal axis of the common holding component (6) is substantially perpendicular to the longitudinal axes of the first and the second piston rod (31, 41).

11. Device (9) according to any of the preceding claims, wherein the device (9) further has an activation component (8) which is in mechanical operative connection with the common holding component (6), and wherein a movement of the activation component (8) leads to the movement of the common holding component (6), preferably wherein a longitudinal axis of the activation component (8) is substantially perpendicular to the longitudinal axis of the common holding component (6), and wherein a direction of movement of the activation component (8) preferably extends substantially perpendicularly to the direction of movement of the common holding component (6).

12. Device (9) according to any of the preceding claims, wherein the conduit apparatus (5) has a first connection element (51) for a fluid connection with the first container (1) and a second connection element (52) for a fluid connection with the second container (2), and wherein each connection element (51, 52) is preferably designed as a needle-like projection for penetrating a septum (12, 22) of the relevant container (1, 2).

13. Device (9) according to any of the preceding claims, wherein the conduit apparatus (5) has a fluid connection with the injection apparatus, and/or wherein the conduit apparatus (5) preferably has a valve element (54), more preferably a one-way valve (54), in particular a check valve (54).

14. Device (9) according to any of the preceding claims, wherein the first container (1) has a first container end and a second container end, and the second container (2) has a first container end and a second container end, wherein the first and the second piston (3, 4) can be pushed between the relevant first container end and the relevant second container end, and wherein contents of the first container (1) can be dispensed via the injection apparatus by pushing the first piston (3) against the first container end of the first container (1).

15. Device (9) according to any of the preceding claims, wherein
each container (1,2) is cylindrical, and/or
the second container (2) is parallel to, in particular in the same direction as, the first container (1) in the device (9).

16. Method for providing a mixed substance using a device (9) according to any preceding claim, comprising a step of moving the activation component (8), which is in operative connection with the common holding component (6), along its longitudinal axis, as a result of which the common holding component (6) is moved substantially in its longitudinal direction, in particular perpendicularly to the direction of movement of the activation component (8), and thereby first releases the engagement between the common holding component (6) and the free end (311) of the first piston rod (31) and subsequently releases the engagement between the common holding component (6) and the free end (411) of the second piston rod (41), preferably wherein the release of the free end (311) of the first piston rod (31) due to the corresponding spring element (32) causes a fluid connection between the conduit apparatus (5) and the first container (1), and the subsequent release of the free end (411) of the second piston rod (41) due to the corresponding spring element (42) causes a fluid connection between the conduit apparatus (5) and the second container (2) and then the second substance (B) is transferred from the second container (2), via the conduit apparatus (5), into the first container (1).

17. Method according to claim 16, wherein the first substance (A) is a solid, in particular a lyophilizate, and the second substance (B) is a liquid, and wherein the liquid is transferred into the first container (1) by pushing the second piston (4) against the first container end of the second container (2), the liquid is combined with the solid in the first container and they form a solution.

## Revendications

1. Dispositif (9) permettant d'administrer au moins une substance, en particulier à un patient, comprenant
un premier récipient (1) contenant une première substance (A) et comportant un premier piston (3) comportant une première tige de piston (31),
un second récipient (2) contenant une seconde substance (B) et comportant un second piston (4) comportant une seconde tige de piston (41),
un appareil d'injection, et
un appareil de conduite (5) permettant de conduire le contenu du second récipient (2) au premier récipient (1) pour y être mélangé et de conduire le contenu du premier récipient (1) à l'appareil d'injection,
dans lequel le premier et le second récipient (2) sont disposés de manière à pouvoir se déplacer dans le dispositif (9) pour l'établissement d'une liaison fluidique avec l'appareil de conduite (5) par l'intermédiaire d'un premier et d'un second élément de raccordement (51, 52) respectif,
dans lequel le premier et le second piston (3, 4) peuvent être déplacés respectivement par l'intermédiaire d'au moins un élément formant ressort (32, 34, 42) précontraint,
dans lequel une extrémité libre (311) de la première tige de piston (31) et une extrémité libre (411) de la seconde tige de piston (41) sont maintenues de manière amovible par un composant de retenue commun (6), et
dans lequel le composant de retenue commun (6) est conçu de telle sorte qu'un déplacement du composant de retenue commun (6) entraîne une libération subséquente de la première tige de piston (31) et de la seconde tige de piston (41) et un déplacement de la première tige de piston (31) et de la seconde tige de piston (41) sensiblement perpendiculairement à la direction de déplacement du composant de retenue commun (6), dans lequel, successivement, par le biais du déplacement de la première tige de piston (31), le premier récipient (1) est amené en liaison fluidique, par l'intermédiaire du premier élément de raccordement (51) et par l'intermédiaire de l'appareil de conduite (5), avec le second récipient (2) par l'intermédiaire du second élément de raccordement (52) par le biais du déplacement subséquent de la seconde tige de piston (41), dans lequel la première substance (A) est mélangée avec la seconde substance (B), dans lequel l'appareil de conduite (5) comprend une vanne unidirectionnelle (54) qui permet l'écoulement de fluide du second récipient (2) dans le premier récipient (1), mais empêche un reflux de fluide du premier récipient (1) dans le second récipient (2).

2. Dispositif (9) selon la revendication 1, dans lequel le composant de retenue commun (6), lequel est de préférence sensiblement en forme de plaque, retient la première tige de piston (31) et la seconde tige de piston (41) à l'encontre de la précontrainte de l'élément formant ressort (32, 42) précontraint respectif.

3. Dispositif (9) selon l'une des revendications précédentes, dans lequel
le composant de retenue commun (6) présente au moins un évidement (61) pour l'extrémité libre (311) de la première tige de piston (31) et au moins un évidement (62) pour l'extrémité libre (411) de la seconde tige de piston (41),
l'extrémité libre (311) de la première tige de piston (31) et l'extrémité libre (411) de la seconde tige de piston (41) présentent respectivement un renfoncement (312, 412) qui est en prise de manière amovible avec le composant de retenue commun (6), et
une taille de l'extrémité libre (311, 411) de la première et de la seconde tige de piston (31, 41) ainsi qu'une taille de l'évidement (61, 62) respectif et/ou une profondeur du renfoncement (312, 412) respectif sont choisies de telle sorte que les extrémités libres (311, 411) de la première et de la seconde tige de piston (31, 41) coopèrent différemment avec le composant de retenue commun (6).

4. Dispositif (9) selon la revendication 3, dans lequel
l'évidement (61) dans le composant de retenue commun (6) pour l'extrémité libre (311) de la première tige de piston (31) présente sensiblement une forme triangulaire et l'évidement (62) dans le composant de retenue commun (6) pour l'extrémité libre (411) de la seconde tige de piston (41) présente sensiblement une forme semi-circulaire, et l'extrémité libre (311) de la première tige de piston (31) présente sensiblement une forme triangulaire et l'extrémité libre (411) de la seconde tige de piston (41) présente sensiblement une forme semi-circulaire, et/ou
la profondeur du renfoncement (412) dans l'extrémité libre (411) de la seconde tige de piston (41) est plus profonde que la profondeur du renforcement (312) dans l'extrémité libre (311) de la première tige de piston (31).

5. Dispositif (9) selon l'une des revendications précédentes, dans lequel l'au moins un élément formant ressort (32) précontraint du premier piston (3), après une libération de la première tige de piston (31), déplace le premier piston (3) conjointement avec le premier récipient (1) par rapport au dispositif (9), et l'au moins un élément formant ressort (42) précontraint du second piston (4), après une libération de la seconde tige de piston (41), déplace le second piston (4) conjointement avec le second récipient (2) par rapport au dispositif (9) et déplace ensuite le second piston (4) par rapport au second récipient (2).

6. Dispositif (9) selon l'une des revendications précédentes, dans lequel la première substance (A) est différente de la seconde substance (B).

7. Dispositif (9) selon l'une des revendications précédentes, dans lequel la seconde substance (B) est un liquide, de préférence dans lequel, en faisant coulisser le second piston (4) contre une extrémité de récipient du second récipient (2), le liquide est transféré dans le premier récipient (1), le liquide s'y mélange avec la première substance (A) et ceux-ci forment une solution, et dans lequel la solution dans le premier récipient (1) peut être administrée par l'intermédiaire de l'appareil d'injection en faisant coulisser le premier piston (3) contre une extrémité de récipient du premier récipient (1).

8. Dispositif (9) selon la revendication 7, dans lequel la première substance (A) est un solide, en particulier un lyophilisat.

9. Dispositif (9) selon l'une des revendications précédentes, dans lequel la libération de l'extrémité libre (311) de la première tige de piston (31) établit une liaison fluidique entre l'appareil de conduite (5) et le premier récipient (1) en raison de l'élément formant ressort (32) correspondant, et dans lequel une libération ultérieure de l'extrémité libre (411) de la seconde tige de piston (41) établit une liaison fluidique entre l'appareil de conduite (5) et le second récipient (2) en raison de l'élément formant ressort (42) correspondant et provoque en outre un transfert de la seconde substance (B) du second récipient (2) dans le premier récipient (1) par l'intermédiaire de l'appareil de conduite (5).

10. Dispositif (9) selon l'une des revendications précédentes, dans lequel un axe longitudinal du composant de retenue commun (6) est sensiblement perpendiculaire aux axes longitudinaux de la première et de la seconde tige de piston (31, 41).

11. Dispositif (9) selon l'une des revendications précédentes, dans lequel le dispositif (9) comprend en outre un composant d'activation (8) qui est en liaison active mécanique avec le composant de retenue commun (6), et dans lequel un déplacement du composant d'activation (8) conduit au déplacement du composant de retenue commun (6), de préférence, dans lequel un axe longitudinal du composant d'activation (8) est disposé sensiblement perpendiculairement à l'axe longitudinal du composant de retenue commun (6), et dans lequel une direction de déplacement du composant d'activation (8) est de préférence sensiblement perpendiculaire à la direction de déplacement du composant de retenue commun (6).

12. Dispositif (9) selon l'une des revendications précédentes, dans lequel l'appareil de conduite (5) présente un premier élément de raccordement (51) pour une liaison fluidique avec le premier récipient (1) et un second élément de raccordement (52) pour une liaison fluidique avec le second récipient (2), et dans lequel chaque élément de raccordement (51, 52) est réalisé de préférence sous la forme d'une saillie en forme d'aiguille destinée à pénétrer dans un septum (12, 22) du récipient (1, 2) respectif.

13. Dispositif (9) selon l'une des revendications précédentes, dans lequel l'appareil de conduite (5) présente une liaison fluidique avec l'appareil d'injection, et/ou dans lequel l'appareil de conduite (5) présente de préférence un élément formant soupape (54), plus préférablement une soupape unidirectionnelle (54), en particulier une soupape antiretour (54).

14. Dispositif (9) selon l'une des revendications précédentes, dans lequel le premier récipient (1) présente une première extrémité de récipient et une seconde extrémité de récipient, et le second récipient (2) présente une première extrémité de récipient et une seconde extrémité de récipient, dans lequel le premier et le second piston (3, 4) peuvent coulisser entre la première extrémité de récipient respective et la seconde extrémité de récipient respective, et dans lequel, au moyen du coulissement du premier piston (3) contre la première extrémité de récipient du premier récipient (1), un contenu du premier récipient (1) peut être administré par l'intermédiaire de l'appareil d'injection.

15. Dispositif (9) selon l'une des revendications précédentes, dans lequel
chaque récipient (1, 2) est réalisé sous forme cylindrique, et/ou
le second récipient (2) est disposé parallèlement au premier récipient (1) dans le dispositif (9), en particulier dans le même sens.

16. Procédé permettant de fournir une substance mélangée avec un dispositif (9) selon l'une des revendications précédentes, comprenant une étape de déplacement du composant d'activation (8) en liaison active avec le composant de retenue commun (6) le long de son axe longitudinal, moyennant quoi le composant de retenue commun (6) est déplacé sensiblement dans sa direction longitudinale, en particulier perpendiculairement à la direction de déplacement du composant d'activation (8), et libère ainsi d'abord la prise entre le composant de retenue commun (6) et l'extrémité libre (311) de la première tige de piston (31) et ensuite la prise entre le composant de retenue commun (6) et l'extrémité libre (411) de la seconde tige de piston (41), de préférence, dans lequel la libération de l'extrémité libre (311) de la première tige de piston (31) provoque une liaison fluidique entre l'appareil de conduite (5) et le premier récipient (1) en raison de l'élément formant ressort (32) correspondant, et la libération subséquente de l'extrémité libre (411) de la seconde tige de piston (41) provoque une liaison fluidique entre l'appareil de conduite (5) et le second récipient (2) en raison de l'élément formant ressort (42) correspondant, et la seconde substance (B) est ensuite transférée du second récipient (2) dans le premier récipient (1) par l'intermédiaire de l'appareil de conduite (5).

17. Procédé selon la revendication 16, dans lequel la première substance (A) est un solide, en particulier un lyophilisat, et la seconde substance (B) est un liquide, et dans lequel, au moyen du coulissement du second piston (4) contre la première extrémité de récipient du second récipient (2), le liquide est transféré dans le premier récipient (1), le liquide s'y combine avec le solide et ceux-ci forment une solution.
